(19) Europäisches Patentamt
European Patent Office
Office européen des brevets

(11) EP 2 641 593 A2

(12) EUROPEAN PATENT APPLICATION

(43) Date of publication:
25.09.2013 Bulletin 2013/39

(21) Application number: 13160634.5

(22) Date of filing: 27.02.2009

(51) Int Cl.:
*A61K 9/20* (2006.01) *A61J 3/10* (2006.01)
*A61K 31/426* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **28.02.2008 US 32145 P**

(62) Document number(s) of the earlier application(s) in accordance with Art. 76 EPC:
**12151574.6 / 2 444 070**
**09714082.6 / 2 257 279**

(71) Applicant: **AbbVie Inc.**
**North Chicago, IL 60064 (US)**

(72) Inventors:
- **Gopinathan, Nishanth**
**Wadsworth, IL Illinois 60083 (US)**
- **Schroeder, Rudolf**
**67551 Worms (DE)**
- **Santiago, Albert**
**Beach Park, IL Illinois 60087 (US)**
- **Faitsch, Lynn V.**
**Libertyville, IL Illinois 60048 (US)**
- **Wardrop, Jacqueline**
**Wilmette, IL Illinois 60091 (US)**
- **Morris, John B.**
**Grayslake, IL Illinois 60030 (US)**
- **Bultmann, Martin**
**68723 Oftersheim (DE)**
- **Schlayer, Heinz**
**67251 Freinsheim (DE)**

(74) Representative: **Modiano, Micaela Nadia et al**
**Modiano & Partners (DE)**
**Thierschstrasse 11**
**80538 München (DE)**

Remarks:
•This application was filed on 22-03-2013 as a divisional application to the application mentioned under INID code 62.
•Claims filed after the date of receipt of application (Rule 68(4) EPC).

# (54) Tablets

(57) The present invention relates to tablets comprising a solid dispersion of at least one therapeutic agent in a matrix,

EP 2 641 593 A2

## Description

This application claims the benefit of US Provisional Application Number 61/032145, filed on February 28, 2008, the entire content of which is incorporated herein by reference.

FIELD OF THE INVENTION

The present invention relates to tablet dosage forms and processes of making the same.

BACKGROUND

A typical method of making tablets involves compressing a mixture of active pharmaceutical ingredient(s) and excipient(s) in a die or mold to give the tablet the desired shape and hardness. A common mechanical unit in tablet compression equipment includes a lower punch, which fits into the die from the bottom, and an upper punch, which enters the die cavity from the top. The tablet is compressed by pressure applied on punches.

Lubricants can be added to the pre-tabletting mixture to help reduce the frictional wear of the die and its associated parts. Binders can also be added to help promote the adhesion of different ingredients in the mixture. Disintegrants help the tablet to disintegrate *in vivo* and thus help deliver the active pharmaceutical ingredient(s) contained in the tablet. A tablet can also be made by tightly compressing active pharmaceutical ingredient(s) without using any excipients.

It is sometimes desirable to coat the tablet. The coating may, for example, be a thin film to prevent decomposition of the tablet. The film may also have other purposes such as to mask a tablet’s unpleasant taste or to delay the disintegration or dissolution of a tablet.

A more detailed description of tablets and their preparation can be found in E. Rudnic and J. Schwartz, "Oral Solid Dosage Forms," REMINGTON’S PHARMACEUTICAL SCIENCE (18th edition, 1990), chapter 89, pp. 1633-1658, the entire content of which is incorporated herein by reference.

SUMMARY OF THE INVENTION

The present invention features processes of making tablets in a lubricated die. The present invention is based on an unexpected finding that a polymer-containing, pre-tabletting material, when blended with lubricants and compressed in an unlubricated die, may form tablets containing internal fractures. These internal fractures are precursors to development of major tablet quality defects such as capping or lamination. The use of a lubricated die surprisingly reduces or eliminates these internal fractures, thereby creating tablets having improved structural stability and physical integrity.

Using a lubricated die also reduces or eliminates internal fractures in tablets which arc made from pre-tabletting materials that arc not blended with any lubricants before compression. Therefore, the present invention features a tabletting process that does not require lubricant blending before compression, Under-blending and over-blending are common issues associated with the lubricant blending process. For instance, over-blending can lead to a physical alignment or "coating" of blended particles with a layer of lubricant. This can lead to delayed dissolution profiles, reduced tablet hardness, increased coating defects, or deteriorated *in vivo* results. The use of a lubricated die allows for the elimination of the lubricant blending process while improving the physical integrity and product quality of the resulting tablets. By eliminating lubricant blending, the new process reduces operation steps, which in turn can improve manufacturing cycle time, simplify implementation of a continuous manufacturing process, enhance process throughput, and reduce manufacturing costs. Moreover, the improved physical integrity of the resulting tablets can reduce the potential for further development of tablet defects during the more robust downstream operations such as coating and bin transport.

In one aspect, the present invention features a process of making tablets, the process comprising the steps of:

compressing a pre-tabletting material in a die to form a tablet, wherein at least one
internal surface of the die is lubricated with at least one lubricant, and the pre-tabletting material comprises at least one therapeutic agent and at least one pharmaceutically acceptable polymer; and
ejecting the tablet from the die.

The pre-tabletting material does not include (1) any therapeutic agent that is denaturalized or inactivated when compressed at a pressure of greater than or equal to 1 ton/$cm^2$, (2) any low molecule active ingredient the elution of which is delayed when compressed at a pressure of greater than or equal to 1 ton/$cm^2$, or (3) any therapeutic agent that is affected by said at least one lubricant.

Preferably, the process does not include any lubricant blending step. The pre- tabletting material also preferably does not comprise any lubricant, or contains only an insignificant amount of lubricant (s) . In many cases, the pre-tabletting material comprises less than 0.5% by weight of lubricant (s) . More preferably, the pre- tabletting material comprises less than 0.2%, 0.1%, 0.05%, 0.01% or lesser by weight of lubricant (s) .

The pre-tabletting material can comprise, for example and without limitation, at least 30% by weight of said at least one pharmaceutically acceptable polymer (including a blend of two or more pharmaceutically acceptable polymers). For instance, the pre-tabletting material can comprise at least 40%, 50%, 60%, 70% or 80% by weight of said at least one pharmaceutically acceptable polymer. The present invention also contemplates the use of pre-tabletting materials that contain less than 30% by weight of said at least one pharmaceutically acceptable polymer. For instance, the pre-tabletting material can comprise at least 25%, 20%, 15% or 10% by weight of said at least one pharmaceutically acceptable polymer.

In one embodiment, each of said at least one pharmaceutically acceptable polymer is hydrophilic (or water-soluble). Preferably, each of said at least one pharmaceutically acceptable polymer has a Tg of at least 50°C. Non-limiting examples of suitable polymers include homopolymers or copolymers of N-vinyl pyrrolidone, such as copovidone, or hydroxypropyl methylcellulose (HPMC).

Polymers that arc water-insoluble or poorly water-soluble can also be used.

Any suitable lubricant can be used in the present invention. A non-limiting example of a preferred lubricant is sodium stearyl fumarate.

Where the pre-tabletting material is compressed by a lower punch and an upper punch, the upper surface of the lower punch and/or the lower surface of the upper punch can also be lubricated. The die and the punches can be lubricated with the same lubricant(s) or different lubricants.

In one embodiment, the therapeutic agent(s) comprised in the pre-tabletting material is formulated as a solid dispersion in a matrix which comprises said at least one pharmaceutically acceptable polymer. Where the pre-tabletting material comprises two or more therapeutic agents, the agents can be formulated in the same solid dispersion or different solid dispersions.

The matrix can further comprise at least one pharmaceutically acceptable surfactant. In one embodiment, each of said at least one pharmaceutically acceptable surfactant has an HLB value of from 4 to 10. Preferably, said at least one pharmaceutically acceptable surfactant comprises a sorbitan mono fatty acid ester, such as sorbitan monolaurate.

In one embodiment, the pre-tabletting material comprises ritonavir, or a combination of ritonavir and lopinavir. The tablet made from this pre-tabletting material preferably shows a dose-adjusted AUC of ritonavir plasma concentration in dogs, under non-fasting conditions, of at least 5 $\mu g \cdot h/ml/100$ mg, and/or a dose-adjusted AUC of lopinavir plasma concentration in dogs, under non-fasting conditions, of at least 15 $\mu g \cdot h/ml/100$ mg. In another embodiment, the pre-tabletting material comprises ritonavir and another therapeutic agent which is metabolized by cytochrome P450 oxidase (e.g., CYP3A4) or transported by P-glycoprotein.

In still another embodiment, the present invention features a process of making tablets, the process comprising the steps of:

compressing a pre-tabletting material in a die to form a tablet, wherein an internal
surface of the die is lubricated with at least one lubricant, and the pre-tabletting material comprises a solid dispersion of at least one therapeutic agent in a matrix, said matrix comprising at least one pharmaceutically acceptable polymer, and said at least one therapeutic agent comprising ritonavir; and
ejecting the tablet from the die.

The pre-tabletting material can comprise, for example, at least 30%, 40%, or 50% by weight of said at least one pharmaceutically acceptable polymer. Preferably, each of said at least one pharmaceutically acceptable polymer is hydrophilic (or water-soluble) and has a Tg of at least 50°C. More preferably, said at least one pharmaceutically acceptable polymer comprises copovidone. The matrix may further comprise at least one pharmaceutically acceptable surfactant. Without limitation, each of said at least one pharmaceutically acceptable surfactant preferably has an HLB value of from 4 to 10. A non-limiting example of a preferred surfactant is sorbitan monolaurate.

The pre-tabletting material employed in the present invention can be prepared by a variety of means known in the art, such as melt-extrusion, spray drying, freeze drying, solvent evaporation, wet granulation, dry granulation, direct blend, and fluidized bed granulation. One of the preferred methods is melt extrusion which comprises the steps of:

solidifying a melt comprising the therapeutic agent(s) and said at least one pharmaceutically acceptable polymer; and
milling the solidified melt to provide the pre-tabletting material.

The present invention also features a tabletting process using a pre-tabletting material that has reduced particle sizes. Where a pre-tabletting material contains granules of small sizes and is compressed at a relatively low pressure, the resulting tablets surprisingly contain significantly less or no detectable internal fractures, even when the compression is carried out in an unlubricated die and using unlubricated punches. Accordingly, in another aspect, the present invention

features another process of making tablets, the process comprising the steps of:

compressing a granular or powdery material in a die to form a tablet, wherein said granular or powdery material comprises at least one therapeutic agent and at least one pharmaceutically acceptable polymer, and at least 90% of particles in said granular or powdery material are smaller than 400 μm; and ejecting said tablet from said die.

In one embodiment, 90% of the particles in the granular or powdery material is smaller than 300 μm, such as smaller than 200 μm, or smaller than 100 μm. The present invention also contemplates the use of a pre-tabletting material where the mean particle size of the pre-tabletting material is no greater than 200 μm, preferably no greater than 150 μm, and more preferably no greater than 100 μm. Any pre-tabletting material described herein can be subject to particle size reduction and then used to make tablets with reduced or eliminated internal fractures.

Furthermore, the present invention features tablets made according to the processes of the present invention. In one embodiment, the tablet comprises a solid dispersion of at least one therapeutic agent in a matrix, wherein the matrix comprises at least one pharmaceutically acceptable hydrophilic polymer (or at least one pharmaceutically acceptable water- soluble polymer) and optionally, at least one pharmaceutically acceptable surfactant, wherein the tablet does not contain any lubricant, or the total amount of lubricant or lubricants in the tablet is less than 0.5% by weight of the tablet, and wherein the tablet does not contain (1) any therapeutic agent that is denaturalized or inactivated when compressed at a pressure of greater than or equal to I ton/$cm^2$, (2) any low molecule active ingredient the elution of which is delayed when compressed at a pressure of greater than or equal to 1 ton/$cm^2$, or (3) any therapeutic agent that is affected by said lubricant or lubricants. In many cases, the tablet does not contain any lubricant, or the total amount of lubricant or lubricants in the tablet is less than 0.25%, 0.1%, 0.05%, 0.01% or lesser by weight of the tablet. The therapeutic agent (s) formulated in the tablet may comprise, for example and without limitation, ritonavir, or a combination of ritonavir and another therapeutic agent (e.g., a combination of ritonavir and lopinavir, or a combination of ritonavir and another therapeutic agent that is metabolized by CYP3A4 or transported by P- glycoprotein) .

In another embodiment, a tablet made according to the present invention comprises a compressed core which includes a solid dispersion of at least one therapeutic agent in a matrix, wherein the matrix comprises at least one pharmaceutically acceptable hydrophilic polymer (or at least one pharmaceutically acceptable water-soluble polymer) and optionally, at least one pharmaceutically acceptable surfactant, and wherein the core does not contain any lubricant therein, and the tablet does not contain (1) any therapeutic agent that is denaturalized or inactivated when compressed at a pressure of greater than or equal to 1 ton/$cm^2$, (2) any low molecule active ingredient the elution of which is delayed when compressed at a pressure of greater than or equal to 1 ton/$cm^2$, or (3) any therapeutic agent that is affected by a lubricant comprised In said tablet (if any). The core can be coated with a film coat. Residual lubricant(s) may be detectable in the tablet as a result of contamination from lubricated die and/or punches. The therapeutic agent(s) formulated in the tablet may comprise, for example and without limitation, ritonavir, or a combination of ritonavir and another therapeutic agent (e.g., a combinatian of ritonavir and lopinavir, or a combination of ritonavir and another therapeutic agent that is metabolized by CYP3A4 or transported by P-glycoprotein).

Other features, objects, and advantages of the present invention are apparent in the detailed description that follows. It should be understood, however, that the detailed description, while indicating preferred embodiments of the invention, are given by way of illustration only, not limitation. Various changes and modifications within the scope of the invention will become apparent to those skilled in the art from the detailed description.

BRIEF DESCRIPTION OF THE DRAWINGS

The drawings are provided for illustration, not limitation.

Figure 1 schematically depicts a 4-step tabletting cycle comprising on-line lubrication (Step 1), die filling (Step 2), tablet compression (Step 3), and tablet ejection (Step 4).

Figure 2A shows the particle size distribution of the target milled extrudate.

Figure 2B illustrates the particle size distribution of the fine extrudate, which was separated from the coarse extrudate by sieving the target milled extrudate using 170 micron mesh.

Figure 2C depicts the particle size distribution of the coarse extrudate.

Figure 3 illustrates the mean yield pressure for different formulations.

Figure 4A demonstrates the X-ray microtomography (XMT) image of a tablet made of the target milled extrudate and compressed at 36 kN load without die wall lubrication.

Figure 4B shows the XMT image of a tablet made of the target milled extrudate and compressed at 42 kN load without die wall lubrication.

Figure 4C describes the XMT image of a tablet made of the target milled extrudate and compressed at 48 kN load without die wall lubrication.

Figure 4D shows the XMT image of a tablet made of the target milled extrudate and compressed at 42 kN load with die wall lubrication.

Figure 4E demonstrates the XMT image of a tablet made of the target milled extrudate and compressed at 48 kN load with die wall lubrication.

Figure 5A shows the XMT images of a tablet made of the fine extrudate and compressed at 36 kN without die wall lubrication.

Figure 5B illustrates the XMT images of a tablet made of the fine extrudate and compressed at 36 kN with die wall lubrication.

Figure 6A shows the XMT images of a tablet made of the fine extrudate and compressed at 42 kN without die wall lubrication.

Figure 6B depicts the XMT images of a tablet made of the fine extrudate and compressed at 42 kN with die wall lubrication.

Figure 7A illustrates the XMT images of a tablet made of the fine extrudate and compressed at 48 kN without die wall lubrication.

Figure 7B illustrates the XMT images of a tablet made of the fine extrudate and compressed at 48 kN with die wall lubrication.

Figure 8A shows the XMT images of a tablet made of the coarse extrudate and compressed at 42 kN without die wall lubrication.

Figure 8B shows the XMT images of a tablet made of the coarse extrudate and compressed at 42 with die wall lubrication.

Figure 9 depicts the XMT images of a tablet made of the coarse extrudate and compressed at 48 kN with die wall lubrication.

## DETAILED DESCRIPTION

The present invention features tablets having reduced or eliminated internal fractures. The present invention also features tabletting processes including the step of compressing a pre-tabletting material in a lubricated die, This aspect of the invention is based on an unexpected finding that a polymer-containing, pre-tabletting material, when compressed at high pressures in an unlubricated die, forms tablets containing internal fractures that may cause mechanical failures such as capping and/or delamination. When the same material (either blended or not blended with lubricants) is compressed in a lubricated die, the resulting tablets contain significantly less or no detectable internal fractures. By reducing the particle sizes of the pre-tabletting material, the present invention also surprisingly found that the resulting tablets contain significantly less or no detectable internal fractures.

Capping and delamination are defects that are often not discovered until coating or packaging. The present invention used a non-invasive method to predict the capping or delamination tendency of a tablet. The method employs X-ray microtomography (XMT) to image the internal structure of the tablet. The presence of internal fractures in the tablet is often indicative of an increased risk of capping or lamination. Using XMT, it was unexpectedly found that when a polymer-containing, pre-tabletting material is compressed in an unlubricated die, the resulting tablets contain a notable amount of internal fractures, which substantially increase the capping or delamination risk of the tablets. It was surprisingly found that by lubricating the die wall before compression, the internal fractures in the tablets could be significantly reduced or eliminated, leading to improved physical integrity and physical stability of the tablets.

Accordingly, the present application features a process of making tablets comprising the steps of:

compressing a pre-tabletting material in a die to form a tablet, wherein an internal surface of the die is lubricated with at least one lubricant, and the pre-tabletting material comprises at least one therapeutic agent and at least one pharmaceutically acceptable polymer; and
releasing or ejecting the tablet from the die.

The lubricated internal surface of the die is in contact with the pre-tabletting material or the tablet at least at one point during compression or ejection. In many instances, the lubricated internal surface of the die is in contact with the pre-tabletting material throughout tablet compression, or is In contact with the tablet throughout tablet ejection. Preferably, the entire internal surface of the die that is in contact with the pre-tabletting material and the tablet during compression and ejection is lubricated.

A common mechanical unit in tablet compression equipment includes two punches which operate within the die cavity. The pre-tabletting material, often in granular or powdery forms, is filled into the die cavity and then compressed by the punches under a predetermined pressure to form a tablet. The surfaces of the punches that are In contact with the pre-tabletting material during compression are herein referred to as the compression surfaces. The compression surface(s) can be engraved or embossed with symbols, initials, or other design patterns. Where the two punches are

aligned vertically, they are referred to as the lower punch and the upper punch, respectively, depending on their locations relative to the die. The lower punch enters the die from the bottom, and the upper punch enters the die from the top. The compression surface of the lower punch is herein referred to as the upper surface of the lower punch, and the compression surface of the upper punch is herein referred to as the lower surface of the upper punch.

According to the present invention, at least one compression surface of the two punches (e.g., the upper surface of the lower punch, or the lower surface of the upper punch) can also be lubricated before die fill. Preferably, both of the compression surfaces (e.g., both the upper surface of the lower punch and the lower surface of the upper punch) are lubricated.

In one embodiment, the internal surface of the die that is contact with the pre-tabletting material during compression and/or the tablet during ejection, as well as the compression surfaces of the two punches (e,g., the upper surface of the lower punch and the lower surface of the upper punch), are lubricated before the pre-tabletting material is filled into the die. These surfaces can be lubricated with the same lubricant(s). They can also be lubricated with different lubricants. The pre-tabletting material is preferably in contact with the lubricated surfaces throughout the duration of compression, and the tablet made therefrom is preferably in contact with the lubricated die wall and the lubricated compression surface of the ejection punch during the course of ejection.

Lubrication of the internal surface of the die and/or the compression surfaces of the punches may provide several advantages. Firstly, lubrication may reduce or eliminate, internal fractures in the tablet, thereby improving the structural stability of the tablet. This may in turn increase the process yield by reducing product loss due to capping. Secondly, lubrication of the die wall and/or compression surfaces may eliminate the necessity of adding lubricant(s) to the pre-tabletting material, thereby reduce operation steps. This could also eliminate the dependence of final tablet quality on the execution of final blend step (for example, control may be no longer needed for the blending time, order of powder(s) addition to the blender, speed of the blender, or number of revolutions). The removal of an extra operation step can potentially improve manufacturing code time, simplify continuous manufacturing, enhance process throughput, and reduce manufacturing costs. These improvements in process may also lead to increased batch size and reduced cost for analytical release testing. Furthermore, die wall lubrication enables the use of lower compression pressures to make tablets and, therefore, can reduce wear and tear of the tabletting machine (including punches and dies) during each operation cycle.

Lubricants suitable for the present invention include, but are not limited to, fatty acids (e.g., stearic acid) ; fatty acid salts (e.g., stearic acid salts such as calcium stearate, magnesium stearate, aluminum stearate, zinc stearate, potassium stearate or sodium stearate, or fatty acid sodium salts) ; fatty acid esters (e.g., stearin or other stearic acid esters) ; aliphatic alcohol esters (e.g., sodium stearyl fumarate) ; lauryl sulfate salts (e.g., sodium lauryl sulfate or magnesium lauryl sulfate) ; waxes (e.g., beeswax, carnauba wax, spermaceti, or other animal, plant, mineral, petroleum or synthetic waxes) ; fats; talc; hydrogenated oils; polyethylene glycols; boric acid; adipic acid; fumarlc acid; silicic acid anhydrate or hydrate; hydrated aluminum silicate, sulfate salts or esters (e.g., sodium sulfate) ; magnesium oxide; glycol; sodium benzoate; glyceryl behenate; leucine; starch; powdered gum arabic; aspartame (N- α- L- Aspartyl- L- phenylalanine 1- methyl ester) ; or any mixture thereof. The internal surface of the die and/or the compression surfaces of the punches can be lubricated by one lubricant, or a combination of two or more lubricants, either with or without other additives. Preferably, the internal surface of the die and/or the compression surfaces of the punches are lubricated with sodium stearyl fumarate, or a combination of sodium stearyl fumarate and one or more other lubricants. The lubricant (s) employed in the present invention can be in any suitable physical forms (e.g., solid, semi- solid, paste, liquid, powder, solution, atomized, or any mixture thereof) .

Lubricant (s) can be applied to the internal surface of the die and/or the compression surfaces of the punches by a variety of means. For instance, lubricant (s) can be manually or automatically applied to the die wall and/or compression surfaces using brushes. Lubricant (s) can also be applied to these surfaces by using a dispensing system, such as those described in U.S. Patent Nos. 5, 609, 908, 5, 700, 492, 6, 764, 695, and 6, 964, 779, all of which are incorporated herein by reference in their entireties. Suitable lubricant dispensing systems are also commercially available, such as the PKB 1, PKB 2 and PKB 3 systems manufactured by FETTE GMBH (Germany) .

A lubricant dispensing system can be readily coupled to a tablet compression machine such that the step of lubrication is coordinated with other tabletting steps. See, e.g., the systems described in U.S. Patent Nos. 6,764,695 and 6,964,779, Such a dispensing system becomes an "on-line" or "die-wall" lubrication unit which allows for continuous tabletting cycles. Each cycle typically comprises the steps of lubricating the internal surface of the die and/or the compression surfaces, filling the die with the pre-tabletting material, compressing the pre-tabletting material in the die to form a tablet, and ejecting the tablet from the die. Figure 1 schematically depicts an exemplary tabletting cycle which comprises on-line lubrication (Step 1). In Step 1, a lubricant (or a combination of lubricants) is sprayed through a spray nozzle(s) onto the internal surface of the die and the compression surfaces of the punches. In Step 2, the lubricated unit moves to a filling area (1), where the pre-tabletting material (3) is filled into the die. In Step 3, the pre-tabletting material is compressed under pressure by the punches to form a tablet (4). The tablet is then pushed out of the die by the lower punch in Step 4, and removed from the equipment by a tablet scraper (2). All of these steps can be performed on a

rotary tablet compression machine. Additional steps, such as precompression after Step 2 and before Step 3, can also be included in the cycle.

The compression pressure in Step 3 (and the precompression pressure if any) can range, for example, from 10 $kN/cm^2$ to 50 $kN/cm^2$, Preferably, the compression pressure is at least 10 $kN/cm^2$ (or at least 1 $ton/cm^2$). For instance, the compression pressure can be at least 15, 16, 17, 18, 19, 20 or more $kN/cm^2$, More preferably, the compression pressure is at least 10 $kN/cm^2$ but no greater than 30 $kN/cm^2$, such as no greater than 25 $kN/cm^2$, 20 $kN/cm^2$ or 15 $kN/cm^2$. Highly preferably, the compression pressure is from 10 $kN/cm^2$ to 20 $kN/cm^2$, such as from 10 $kN/cm^2$ to 15 $kN/cm^2$ or from 15 $kN/cm^2$ to 20 $kN/cm^2$.

Tablets compressed in a lubricated die and/or using lubricated punches often contain significantly less, or no detectable, internal fractures, as compared to tablets compressed In an unlubricated die and using unlubricated punches, depending on factors such as the compression forces, particle sizes, or whether internal lubrication is used. As used herein, "significantly less" means that the average number of internal fractures in the tablets made using lubricated die/punches is at least 25%, preferably at least 50%, less than the average number of internal fractures in the tablets made using unlubricated die/punches. Internal fractures in a tablet can be detected by XMT, as described hereinbelow.

Any therapeutic agent that is amenable to tablet formulations can be tabletted according to the present invention, Non-limiting examples of these therapeutic agents include drugs for the treatment of infections, cancers, immune disorders, neurological disorders, neuro-psychiatric disorders, hematopoietic disorders, endocrine disorders, cardiovascular disorders, liver disorders, metabolic disorders, inflammation, or pain. Specific examples of these therapeutic agents include, but are not limited to, antiviral agents, such as HIV protease inhibitors, HIV reverse transcriptase inhibitors, HIV integrase inhibitors, HIV fusion inhibitors, HCV protease inhibitors, and HCV polymerase inhibitors; antibiotics; anticancer agents, such as chemotherapeutic agents, alkylating agents, antimetabolites, plant alkaloids, antitumor antibiotics, hormones, antihormones, kinase inhibitors, and growth factor receptor inhibitors; or immunomodulators, such as anti-inflammatory agents, immunosuppressive agents, and immunostimulatory agents. As used herein, the terms "therapeutic agent," "active pharmaceutical ingredient" and "active ingredient" are used interchangeably.

Preferably, the therapeutic agent (s) tabletted according to the present invention is not denaturalized or inactivated when compressed at a pressure of greater than or equal to 1 $ton/cm^2$ (about 9.8 $kN/cm^2$) and is not affected by the lubricant (s) used on the die wall and compression surfaces. As used herein, a therapeutic agent is denaturalized or inactivated if the agent is destabilized or dissolved, or when the agent is in a crystal form the elution of the agent becomes slow because its crystal is deformed, by the pressure applied at the time of tabletting or by the friction or heat generated during tabletting, Examples of therapeutic agents that can be denaturalized or inactivated by high tabletting pressures are described in U.S. Patent No. 6, 964, 779 (e.g., Tables 3- 5), the entire content of which is incorporated herein by reference. A therapeutic agent is affected by a lubricant (or a lubricated surface) if the agent is colored, decomposed or deteriorated in its potency upon contact with the lubricant (or the lubricated surface) . Examples of agents that can be affected by lubricants are illustrated In U.S. Patent No. 6, 764, 695, the entire content of which is also incorporated herein by reference.

The pre-tabletting material employed in the present invention can comprise, for example, from 0.1 to 50% by weight of a therapeutic agent (or a combination of therapeutic agents). For instance, the pre-tabletting material can comprise from 0.5 to 25%, preferably from 1 to 20%, by weight of a therapeutic agent (or a combination of therapeutic agents). Where the pre-tabletting material includes two or more therapeutic agents, each agent can be present, for example, from 0.5 to 25%, preferably from 1 to 20%, by weight of the total pre-tabletting material. Pre-tabletting material comprising more than 50%, 60% or 70% by weight of therapeutic agent(s) can also be used in the present invention.

In one example, the pre-tabletting material comprises ritonavir. In another example, the pre-tabletting material comprises lopinavir. In yet another example, the pre-tabletting material comprises a combination of ritonavir and lopinavir, In still another example, the pre-tabletting material comprises a combination of ritonavir and lopinavir in a weight ratio of 1:4. Ritonavir and lopinavir are described in U. S. Patent Nos. 5,541,206 and 5,914,332, respectively, both of which are incorporated herein by reference in their entireties.

In a further example, the pre-tabletting material comprises ritonavir and another therapeutic agent whose pharmacokinetics can be improved by ritonavir. In still another example, the pre-tabletting material comprises ritonavir and another therapeutic agent which is transported by P-glycoprotein.

The pre-tabletting material employed in the present invention preferably does not comprise any lubricant, or contains only an insignificant amount of lubricant(s). As used herein, the amount of a lubricant or lubricants in a pre-tabletting material is "insignificant" if the tablet prepared therefrom has no detectable structural difference from a similarly prepared tablet using an otherwise identical pre-tabletting material but without the lubricant or lubricants. In many cases, the pre-tabletting material does not contain any lubricant, or the total amount of lubricant(s) in the pre-tabletting material is less than 0.5% by weight of the pre-tabletting material. More preferably, the total amount of lubricant(s) in the pre-tabletting material is less than 0,2%, 0.1%, 0,05%, 0.01% or lesser by weight of the pre-tabletting material.

The pre-tabletting material employed in the present Invention comprises a pharmaceutically acceptable polymer or a combination of two or more pharmaceutically acceptable polymers. The pre-tabletting material can comprise, for

example and without limitation, at least 30% by weight of a pharmaceutically acceptable polymer or a combination of pharmaceutically acceptable polymers. For instance, the pre-tabletting material can comprise at least 40%, 50%, 60%, 70% or 80% by weight of a pharmaceutically acceptable polymer or a combination of pharmaceutically acceptable polymers. The present invention also contemplates the use of pre-tabletting materials that contain less than 30% by weight of pharmaceutically acceptable polymer(s).

In many embodiments, the pre- tabletting material comprises a solid dispersion of at least one therapeutic agent In a matrix comprising at least one pharmaceutically acceptable polymer. The term "solid dispersion" defines a system in a solid state (as opposed to a liquid or gaseous state) comprising at least two components, wherein one component is dispersed throughout the other component or components. For example, the active ingredient or combination of active ingredients can be dispersed in a matrix comprised of a pharmaceutically acceptable hydrophilic polymer or a combination of pharmaceutically acceptable hydrophilic polymers, or in a matrix comprised of a pharmaceutically acceptable water-soluble polymer or a combination of pharmaceutically acceptable water- soluble polymers. The matrix can also include other components, such as a pharmaceutically acceptable surfactant (s), a flow regulator (s), a disintegrant (s), a plasticizer (s), a stabilizer (s), or a bulking agent (s) or filler (s) . The term "solid dispersion" encompasses systems having small particles, typically of less than 1 $\mu$m in diameter, of one phase dispersed in another phase. When said dispersion of the components is such that the system is chemically and physically uniform or homogenous throughout or consists of one phase (as defined in thermodynamics), such a solid dispersion wiil be called a "solid solution" or a "glassy solution." A glassy solution is a homogeneous, glassy system in which a solute is dissolved in a glassy solvent. Glassy solutions and solid solutions of therapeutic agents are the preferred physical systems employed in the pre- tabletting material. Glassy solutions and solid solutions do not contain any significant amounts of active ingredients in their crystalline or microcrystalline state, as evidenced by thermal analysis (e, g., differential scanning calorimetry), near- infrared analysis, or X- ray diffraction/ scattering analysis. The present invention also contemplates a pre- tabletting material comprising two or more different solid dispersions each of which includes a different therapeutic agent,

The pharmaceutically acceptable polymer(s) employed in the present invention preferably is water-soluble. In some examples, the water-soluble polymer(s) can have an apparent viscosity, when dissolved at 20°C in an aqueous solution at 2 % (w/v), of from I to 5000 mPa·s, preferably of from 1 to 700 mPa·s, and more preferably of from 5 to 100 mPa·s. Crosslinked or other water-insoluble or poorly water-soluble polymers can also be used, either alone or in combination with water-soluble polymers.

Non- limiting examples of pharmaceutically acceptable water- soluble or hydrophilic polymers suitable for the present invention include homopolymers or copolymers of N- vinyl lactams, such as homopolymers or copolymers of N- vinyl pyrrolidone (e.g., polyvinylpyrrolidone (PVP), or copolymers of N- vinyl pyrrolidone and vinyl acetate or vinyl propionate) ; cellulose esters or cellulose ethers, such as alkylcelluloses (e.g., methylcellulose or ethylcellulose), hydroxyalkylcelluloses (e.g., hydroxypropylcellulose), hydroxyalkylalkylcelluloses (e.g., hydroxypropylmethylcellulose), and cellulose phthalates or succinates (e.g., cellulose acetate phthalate and hydroxypropylmethylcellulose phthalate, hydroxypropylmethylcellulose succinate, or hydroxypropylmethylcellulose acetate succinate) ; high molecular polyalkylene oxides, such as polyethylene oxide, polypropylene oxide, and copolymers of ethylene oxide and propylene oxide; polyacrylates or polymethacrylates, such as methacrylic acid/ ethyl acrylate copolymers, methacrylic acid/ methyl methacrylate copolymers, butyl methacrylate/ 2- dimethylaminoethyl methacrylate copolymers, poly (hydroxyalkyl acrylates), and poly (hydroxyalkyl methacrylates) ; polyacrylamides; vinyl acetate polymers, such as copolymers of vinyl acetate and crotonic acid, and partially hydrolyzed polyvinyl acetate (also referred to as partially saponified "polyvinyl alcohol") ; polyvinyl alcohol; oligo- or polysaccharides, such as carrageenans, galactomannans, and xanthan gum; polyhydroxyalkylacrylates; polyhydroxyalkylmethacrylates; copolymers of methyl methacrylate and acrylic acid; polyethylene glycols (PEGs) ; or any mixture thereof.

In one embodiment, the water-soluble/hydrophilic polymer(s) employed In the present invention has a glass transition temperature (Tg) of at least 50°C, such as at least 60°C, 70°C, or 80°C. Preferably, tlre water-soluble/hydrophilic polymer(s) has a Tg of from 80°C to 180°C. Methods for determining Tg values of organic polymers are described in "Introduction to Physical Polymer Science," 2nd Edition by L.H. Sperling, published by John Wiley & Sons, Inc., 1992. The Tg value can be calculated as the weighted sum of the Tg values for homopolymers derived from each of the individual monomers that make up the polymer, i.e., $Tg = \sum W_i X_i$ where $W_i$ is the weight percent of monomer i in the organic polymer, and $X_i$ is the Tg value for the homopolymer derived from monomer i. Tg values for homopolymers may be taken from "Polymer Handbook," 2nd Edition by J. Brandrup and E.H. Immergut, Editors, published by John Wiley & Sons, Inc., 1975.

Water- soluble/ hydrophilic polymers having a Tg of at least 50°C allow for the preparation of solid dispersions that are mechanically stable and, within ordinary temperature ranges, sufficiently temperature stable so that the solid dispersions may be compacted to tablets without, or with only a small amount of, tabletting aids. Water- soluble/ hydrophilic polymers having a Tg of below 50°C may also be used.

The water-soluble/hydrophilic polymer(s) employed in the present invention preferably exists in an amorphous form, or is capable of forming an amorphous matrix, at room temperature (25°C). Non-limiting examples of these water-soluble/hydrophilic polymers include homopolymers of vinylpyrrolidone (e.g., PVP with Fikentscher K values of from 12 to 100, or PVP with Fikentscher K values of from 17 to 30), and copolymers of 30 to 70% by weight of N-vinylpyrrolidone (VP) and 70 to 30% by weight of vinyl acetate (VA) (e.g., a copolymer of 60% by weight VP and 40% by weight VA).

The pre-tabletting material employed in the present invention (or the solid dispersion comprised therein) can further include a pharmaceutically acceptable surfactant or a combination of pharmaceutically acceptable surfactants. The term "pharmaceutically acceptable surfactant" refers to a pharmaceutically acceptable non-ionic surfactant.

In one embodiment, the pre- tabletting material (or the solid dispersion comprised therein) comprises at least one surfactant having a hydrophilic lipophilic balance (HLB) value of from 4 to 10, preferably from 7 to 9. The HLB system (Fiedler, H.B., Encyclopaedia of Excipients, 5th ed., Aulendorf: ECV- Editio- Cantor- Verlag (2002) ) attributes numeric values to surfactants, with lipophilic substances receiving lower HLB values and hydrophilic substances receiving higher HLB values.

Surfactants having an HLB value of from 4 to 10 and suitable for use In the present invention include, but are not limited to, polyoxyethylene alkyl ethers, e.g. polyoxyethylene (3) lauryl ether, polyoxyethylene (5) cetyl ether, polyoxyethylene (2) stearyl ether, polyoxyethylene (5) stearyl ether; polyoxyethylene alkylaryl ethers, e.g. polyoxyethylene (2) nonylphenyl ether, polyoxyethylene (3) nonylphenyl ether, polyoxyethylene (4) nonylphenyl ether, polyoxyethylene (3) octylphenyl ether; polyethylene glycol fatty acid esters, e.g. PEG-200 monolaurate, PEG-200 dilaurate, PEG-300 dilaurate, PEG-400 dilaurate, PEG-300 distearate, PEG-300 dioleate; alkylene glycol fatty acid mono esters, e.g. propylene glycol monolaurate (Lauroglycol®); sucrose fatty acid esters, e.g. sucrose monostearate, sucrose distearate, sucrose monolaurate, sucrose dilaurate; or sorbitan mono fatty acid esters, e.g., sorbitan monataurate (Span® 20), sorbitan monooleate, sorbitan monopalmitate (Span® 40), or sorbitan stearate; or any mixture thereof. The sorbitan mono fatty acid esters are preferred, with sorbitan monolaurate and sorbitan monopalmitate being particularly preferred.

The pre-tabletting material (or the solid dispersion comprised therein) can also include other pharmaceutically acceptable surfactants having an HLB value of below 4 or above 10. These surfactants can be used either alone or in combination with surfactants having an HLB values of from 4 or 10. Non-limiting examples of these surfactants include polyoxyethylene castor oil derivates, e.g. polyoxyethyleneglycerol triricinoleate or polyoxyl 35 castor oil (Cremophor® EL, BASF Corp.) or polyoxyothyleneglycerol oxystearate such as polyethylenglycol 40 hydrogenated castor oil (Cremophor® RH 40) or polyethylenglycol 60 hydrogenated castor oil (Cremophor® RH 60); block copolymers of ethylene oxide and propylene oxide, also known as polyoxyethylene polyoxypropylene block copolymers or polyoxyethylene polypropyleneglycol, such as Poloxamer® 124, Poloxamer® 188, Poloxamer® 237, Poloxamer® 388, Poloxamer® 407 (BASF Wyandotte Corp.); or mono fatty acid esters of polyoxyethylene (20) sorbitan, e.g. polyoxyetltylene (20) sorbitan monooleate (Tween® 80), polyoxyethylene (20) sorbitan monostearate (Tween® 60), polyoxyethylene (20) sorbitan monopalmitate (Tween® 40), and polyoxyethylene (20) sorbitan monolaurate (Tween® 20).

In one embodiment, the pre-tabletting material employed in the present invention (or the solid dispersion comprised therein) contains two or more surfactants, where at least 50 wt % of all surfactants in the pre-tabletting material (or the solid dispersion) has an HLB value of from 4 to 10. Preferably, at least 60, 70, 80, 90, or more wt % of all surfactants in the pre-tabletting material (or the solid dispersion) has an HLB value of from 4 to 10.

Various techniques can be used to make solid dispersions. These techniques include, but are not limited to, melt-extrusion, spray drying, freeze drying, and solvent evaporation. The solid dispersions thus prepared can be directly compressed to tablets if they are already in powder forms, or milled or ground to small particles before being compressed to tablets. Additional ingredients may also be mixed with the solid dispersions before grinding and/or tablet compression.

The melt- extrusion process typically comprises the steps of preparing a melt which includes the active ingredient (s), the polymer (s) and optionally the surfactant (s), and cooling the melt until it solidifies. "Melting" means a transition into a liquid or rubbery state in which it is possible for one component to get embedded, preferably homogeneously embedded, in the other component or components. In many cases, the polymer component (s) will melt and the other components including the active ingredient (s) and surfactant (s) will dissolve in the melt thereby forming a solution. Melting usually involves heating above the softening point of the polymer (s) . The preparation of the melt can take place in a variety of ways. The mixing of the components can take place before, during or after the formation of the melt. For example, the components can be mixed first and then melted or be simultaneously mixed and melted. The melt can also be homogenized in order to disperse the active ingredient (s) efficiently. In addition, it may be convenient first to melt the polymer (s) and then to mix in and homogenize the active ingredient (s) . In one example, all materials except surfactant (s) are blended and fed into an extruder, while surfactant is molten externally and pumped in during extrusion.

In another example, the melt comprises a water-soluble polymer or a combination of water-soluble polymers, and the melt temperature is in the range of from 70 to 250°C, preferably from 80 to 180°C, and highly preferably from 100 to 140°C.

In yet another example, the melt comprises a hydrophilic polymer or a combination of hydrophilic polymers, and the melt temperature is in the range of from 70 to 250°C, preferably from 80 to 180°C, and highly preferably from 100 to 140°C.

The active ingredient(s) can be employed as such, or as a solution or dispersion in a suitable solvent such as

alcohols, aliphatic hydrocarbons, esters or, in some cases, liquid carbon dioxide. The solvent can be removed, e.g. evaporated, upon preparation of the melt.

Various additives can also be included in the melt, for example, flow regulators (e.g., colloidal silica), binders, lubricants, fillers, disintegrants, plasticizers, colorants, or stabitizers (e.g., antioxidants, light stabilizers, radical scavengers, and stabilizers against microbial attack).

The melting and/or mixing can take place in an apparatus customary for this purpose. Particularly suitable ones are extruders or kneaders. Suitable extruders include single screw extruders, intermeshing screw extruders or multiscrew extruders, preferably twin screw extruders, which can be corotating or counterrotating and, optionally, be equipped with kneading disks. It will be appreciated that the working temperatures will be determined by the kind of extruder or the kind of configuration within the extruder that is used. Part of the energy needed to melt, mix and dissolve the components in the extruder can be provided by heating elements. However, the friction and shearing of the material in the extruder may also provide a substantial amount of energy to the mixture and aid in the formation of a homogeneous melt of the components.

The melt can range from thin to pasty to viscous. Shaping of the extrudate can be conveniently carried out by a calender with two counter- rotating rollers with mutually matching depressions on their surface. The extrudate can be cooled and allow to solidify. The extrudate can also be cut into pieces, either before (hot- cut) or after solidification (cold-out) .

The solidified extrusion product can be further milled, ground or otherwise reduced to granules. Like the solidified extrudate, each granule comprises a solid dispersion, preferably a solid solutions, of the active ingredient (s) in a matrix comprised of the polymer (s) and optionally the surfactant (s) . These granules can be directly fed into a die and compacted to a tablet. Alternatively, these granules can be blended with other active ingredient (s) and/or additive (s) before die fill and compaction. These additional additives can be, for example, flow regulators, disintegrants, and bulking agents (fillers) . Disintegrants promote a rapid disintegration of the tablet in the stomach and keeps the granules which are liberated separate from one another. Suitable disintegrants include, but are not limited to, crosslinked polymers such as crosslinked polyvinyl pyrrolidone and crosslinked sodium carboxymethylcellulose, Suitable bulking agents (also referred to as "fillers") can be selected, for example, from lactose, calcium hydrogenphosphate, microcrystalline cellulose (Avicel®), silicates, silicon dioxide, magnesium oxide, talc, potato or corn starch, isomalt, or polyvinyl alcohol. Suitable flow regulators include, but are not limited to, highly dispersed silica (Aerosil®), and animal or vegetable fats or waxes. Other additives can also be added before die fill, for example, dyes such as azo dyes, organic or inorganic pigments such as aluminium oxide or titanium dioxide, or dyes of nature origin; and stabilizers such as antioxidants, light stabilizers, radical scavengers, or stabilizers against microbial attack.

The extrusion product can also be blended with other active ingredient(s) and/or additive(s) before being milled or ground to granules.

Preferably, the granules made from the extrudates are not blended with any lubricants before being fed into a die for compression. More preferably, the granules are directly fed into a die and compacted to a tablet without being blended with any additives or other ingredients.

Spray drying involves breaking up liquid mixtures into small droplets and rapidly removing solvent from the mixture in a container (spray drying apparatus) where there is a strong driving force for evaporation of solvent from the droplets, The strong driving force for solvent evaporation is often provided by maintaining the partial pressure of solvent in the spray drying apparatus well below the vapor pressure of the solvent at the temperatures of the drying droplets. This may be accomplished by either (1) maintaining the pressure in the spray drying apparatus at a partial vacuum; (2) mixing the liquid droplets with a warm drying gas; or (3) both.

The spray drying apparatus suitable for the present invention can be any of the various commercially available apparatus. Non-limiting examples of specific spray drying devices include those manufactured by Niro Inc., Buchi Labortechnik AG, and Spray Drying Systems, Inc. Spray drying processes and spray drying equipment are described generally in Perry's Chemical Engineers' Handbook, Sixth Edition (R. H. Perry, D. W. Green, J. O. Maloney, eds.) McGraw-Hill Book Co. 1984, pages 20-54 to 20-57. More details on spray drying processes and equipment are reviewed by Marshall, "Atomization and Spray Drying," 50 Chem. Eng. Prog. Monogr. Series 2 (1954). The contents of these references are incorporated herein by reference.

Besides melt extrusion and spray drying and other solid dispersion technology, the pre- tabletting material can also be prepared using other methods, such as wet granulation, dry granulation or fluidized bed granulation as described in E. Rudnic and J. Schwartz, "Oral Solid Dosage Forms, " REMINGTON'S PHARMACEUTICAL SCIENCE (18th edition, 1990), chapter 89, pp. 1641- 1645, the entire content of which is incorporated herein by reference. All ingredients in the pre- tabletting material preferably are well granulated and mixed, such that the tablet made therefrom has an even distribution of the active ingredient (s) and the amount of the active ingredient (s) in each tablet is about equal. Where the active ingredient (s), polymer (s), and optionally, surfactant (s) and/or other additives can form a sufficiently homogenous mixture by simple mixing, the simple mixture of these ingredients can be directly used as a pre- tabletting material for die fill and tablet compression (direct compression) .

Granules of various sizes can be compacted to tablets according to the present invention. Preferably, the sizes of the granules in a pre-tabletting material are substantially uniform. The mean particle size of the granules in a pre-tabletting material can range, for example, from 10 μm to 1000 μm. For instance, the mean particle size of the granules can ranges from 50 to 100 μm, from 100 to 200 μm, from 200 to 300 μm, from 300 to 400 μm, from 400 to 500 μm, from 500 to 600 μm, or from 600 to 700 μm. In one embodiment, at least 90% of the granules in a pre-tabletting material are smaller than 700 μm (or smaller than 600, 500, 400, 300, 200, or 100 μm). In another embodiment, at least 90% of the granules in a pre-tabletting material are greater than 10 μm (or greater than 20, 50, 100, 150, or 200 μm). In still another embodiment, at least 75% of the granules in a pre-tabletting material have particle sizes of from 10 to 300 μm (or from 100 to 700 μm, or from 30 to 600 μm).

The particle size distribution can be determined by sieve analysis or other means as appreciated by those skilled in the art, An exemplary sieve analysis involves the use of sleves with different opening sizes. The sieves can be assembled into a column with the top sieve having the widest openings and each lower sieve in the column having smaller openings than the one above. The column can be placed in a mechanical shaker. The shaker shakes the column for a pre-determined amount of time. After the shaking is complete, the material on each sieve is weighed. The weight of the sample of each sieve is then divided by the total weight to give a percentage retained on each sieve. The mean particle size can be calculated using Equation 1:

$$MPS = \frac{C_1 - 50\%}{C_1 - C_2} \times (S_2 - S_1) + S_1 \qquad \text{Equation 1}$$

where $C_1$ is the accumulated percentage of particles just larger than 50%; $C_2$ is the accumulated percentage of particles just smaller than 50%; $S_1$ is the size of particles which has an accumulated percentage just larger than 50%, and $S_2$ is the size of the particles which have an accumulated percentage just smaller than 50%.

Where a pre-tabletting material contains granules of small sizes and is compressed at a relatively low pressure, the resulting tablets may contain significantly less or no detectable internal fractures, even when the compression is carried out in an unlubricated die and using unlubricated punches. Accordingly, the present invention features a process of making tablets from a granular pre-tabletting material, where 90% of the granules in the pre-tabletting material are smaller than 400 μm, preferably smaller than 300 μm, highly preferably smaller than 200 μm, and most preferably smaller than 100 μm. In addition, the present invention features a process of making tablets from a granular pre-tabletting material, where the mean particle size of the granular pre-tabletting material is no greater than 200 μm, preferably no greater than 150 μm, and more preferably no greater than 100 μm. These processes comprise the steps of compressing the pre-tabletting material in a die to form a tablet, and ejecting the tablet from the die. The internal surface of the die or the compression surfaces of the punches can be either lubricated or unlubricated. Preferably, the compression pressure employed in this process is no more than 30 $kN/cm^2$, such as no more than 25, 20 or 15 $kN/cm^2$.

In one aspect, the present invention features the use of a pre- tabletting material which comprises a solid dispersion of at least one therapeutic agent in a matrix, wherein said at least one therapeutic agent comprises (1) ritonavir or (2) ritonavir and another therapeutic agent (e.g., lopinavir or a therapeutic agent which is metabolized by cytochrome P450 oxidase or transported by P- glycoprotein), and said matrix comprises one or more pharmaceutically acceptable hydrophilic polymers (or one or more pharmaceutically acceptable water- soluble polymers) . Preferably, the pre- tabletting material does not include (1) any therapeutic agent that is denaturalized or inactivated when compressed at a pressure of greater than or equal to 1 $ton/cm^2$, (2) any low molecule active ingredient the elution of which is delayed when compressed at a pressure of greater than or equal to 1 $ton/cm^2$, or (3) any therapeutic agent that is affected by the lubricant (s) used on the internal surface of the die and/or the compression surfaces.

The pre-tabletting material employed in this aspect of the invention can comprise, for example, at least 50% by weight of said one or more water-soluble/hydrophilic polymers. Preferably, the pre-tabletting material comprises at least 60%, 65% or 70% by weight of said one or more water-soluble/hydrophilic polymers. Each of said one or more water-soluble/hydrophilic polymers preferably has a Tg of at least 50°C. More preferably, each of said one or more water-soluble/hydrophilic polymers has a Tg of from 80 to 180°C. Water-soluble/hydrophilic polymers with a Tg of below 50°C and/or water-insoluble polymers can also be included In the matrix.

The matrix can further include one or more pharmaceutically acceptable surfactants. The pre-tabletting material can comprise, for example, from 1 to 30% by weight of said one or more surfactants. Preferably, the pre-tabletting material comprises from 2 to 20% by weight of said one or more surfactants. Each of said one or more surfactants preferably has an HLB value of from 4 to 10, Surfactants having an HLB value of below 4 or above 10 may also be used. Where the pre-tabletting material comprises two or more surfactants, preferably at least 50 wt % of all surfactants, based on the total weight of all surfactants in the pre-tabletting material, have an HLB value(s) of from 4 to 10.

Any water- soluble/ hydrophilic polymer and surfactant described hereinabove may be used in this aspect of the invention. Preferred water- soluble/ hydrophilic polymers include homopolymers or copolymers of N- vinyl pyrrolidone,

such as copovidone. Preferred surfactants include sorbitan mono fatty acid esters, such as sorbitan monolaurate. The matrix employed in this aspect of the invention can also include other additive (s), such as colloidal silicon dioxide.

The pre-tabletting material employed in this aspect of the invention preferably is a granular or powdery material which comprises particles, each particle including a solid dispersion of said at least one therapeutic agent in said matrix. The solid dispersion preferably is a solid solution or a glassy solution. The matrix preferably is an amorphous matrix in which said at least one therapeutic agent (e.g., ritonavir, or ritonavir and another therapeutic agent) is molecularly dispersed. More preferably, the pre-tabletting material is prepared by melt extrusion. In one example, the melt extrusion includes the steps of (1) preparing a melt comprising said at least one therapeutic agent, said one or more water-soluble/hydrophilic polymers and, optionally, said one or more surfactants, (2) solidify said melt, and (3) milling or grinding said melt to provide the pre-tabletting material. Spray drying and other methods for the preparation of solid dispersions can also be used to prepare the pre-tabletting material of this aspect of the invention.

The pre-tabletting material employed in this aspect of the invention can comprise, for example, at least 1, 5, 10, 15 or 20% by weight of ritonavir. In many cases, the pre-tabletting material comprises from 1 to 50% by weight of ritonavir. Preferably, the pre-tabletting material comprises from 1 to 20% by weight of ritonavir. In one example, the pre-tabletting material comprises, relative to the total weight of the pre-tabletting material, from 3 to 30 % by weight of ritonavir or ritonavir and another therapeutic agent, from 2 to 20 % by weight of said one or more surfactants, and at least 50% by weight of said one or more water-soluble/hydrophilic polymers, where each said surfactant preferably has an HLB value of from 4 to 10 and each said polymer preferably has a Tg of at least 50°C. Specific examples of pre-tabletting materials suitable for this aspect of the invention can be found in Tables 1 and 2 and Examples 1-7 of U.S. Patent Application Publication No. 20050084529.

The tablet made according to this aspect of the invention preferably shows a dose-adjusted AUC of ritonavir plasma concentration in dogs, under non-fasting conditions, of at least 5 μg·h/ml/100 mg, such as at least 9, 10, 15 or 20 μg·h/ml/100 mg.

The tablet thus made is often characterized by an excellent stability, and exhibits high resistance against recrystallization or decomposition of ritonavir and/or other active ingredient(s). In many cases, upon storage for 6 weeks at 40°C and 75% humidity (e.g., when kept in high density polyethylene (HDPE) bottles without desiccant), the tablet contains at least 98 % of the initial content of ritonavir and other active ingredient(s) (as evidenced by HPLC analysis) and does not exhibit any sign of crystallinity (as evidenced by near infrared spectroscopy, differential scanning calorimetry, or wide-angle x-ray scattering analysis).

In one embodiment of this aspect of the invention, the pre-tabletting material comprises a solid dispersion of ritonavir and lopinavir. The pre-tabletting material can comprise, for example, at least 5, 10, 15, 20, 25, 30, 35 or 40% by weight of lopinavir. Preferably, the pre-tabletting material comprises from 5 to 40% by weight of lopinavir. In one example, the pre-tabletting material comprises from 1 to 10% by weight of ritonavir and from 4 to 40% by weight of lopinavir. The ratio of ritonavir over lopinavir may range, for example, from 1:1 to 1:8, and the preferred ritonavir over lopinavir ratio is 1:4. Non-limiting examples of ritonavir/lopinavir combination include 2% ritonavir and 8% lopinavir, 4% ritonavir and 16% lopinavir, or 8% ritonavir and 32% lopinavir.

Preferably, the tablet made from this pre- tabletting material shows a dose- adjusted AUC of ritonavir plasma concentration in dogs, under non- fasting conditions, of at least 5 μg·h/ml/ 100 mg (e.g., at least 9, 10, 15 or 20 μg·h/ml/ 100 mg), and a dose- adjusted AUC of lopinavir plasma concentration in dogs, under non- fasting conditions, of at least 15 μg·h/ml/ 100 mg (e.g., at least 20, 22.5, 25, 30, 35, 40, 45 or 50 μg·h/ml/ 100 mg) . For instance, the tablet can have a dose- adjusted AUC of ritonavir plasma concentration of at least 5 μg·h/ml/ 100 mg and a dose- adjusted AUC of lopinavir plasma concentration of at least 15 μg·h/ml/ 100 mg; or a dose- adjusted AUC of ritonavir plasma concentration of at least 9 μg·h/ml/ 100 mg and a dose- adjusted AUC of lopinavir plasma concentration of at least 20 μg·h/ml/ 100 mg (preferably at least 22.5 μg·h/ml/ 100 mg, most preferred at least 35 μg·h/ml/ 100 mg) ; or a dose- adjusted AUC of ritonavir plasma concentration of at least 10 μg·h/ml/ 100 mg and a dose- adjusted AUC of lopinavir plasma concentration of at least 40 μg·h/ml/ 100 mg, where all AUCs are measured in dogs under non- fasting conditions.

As used herein, "AUC" refers to the area under the plasma concentration time curve (AUC) from 0 to 24 hours, where the tablet at issue has been administered orally to dogs (beagle) under non-fasting conditions. "Non-fasting condition" means that the dogs receive a nutritionally balanced daily ration during the pre-test period and the whole test period. The AUC has units of concentration times time. Once the experimental concentration-time points have been determined, the AUC may conveniently be calculated, e.g. by a computer program or by the trapezoidal method. All AUC data are dose adjusted to the 100 mg dose level. For the purposes herein, the AUC is preferably determined within a dose range where the AUC increases proportionally with dose. Protocol for the oral bioavailability studies in beagle dogs and the AUC calculations are described in U.S. Patent Application Publication No. 20050084529, the entire content of which is incorporated herein by reference.

In another embodiment of this aspect of the invention, the pre-tabletting material comprises a solid dispersion of ritonavir and another therapeutic agent whose pharmacokinetics can be improved by ritonavir. Ritonavir has been shown to be capable of Inhibiting cytochrome P450 oxidase, such as the P450 3A4 isozyme (CYP3A4). As a result, co-administration of ritonavir with a drug that is metabolized by cytochrome P450 oxidase (e.g., CYP3A4) may enhance the pharmacokinetics of the drug. See U.S. Patent No. 6,037,157, which is incorporated herein by reference in its entirety. As used herein, improved pharmacokinetics refers to improved $C_{max}$ (the maximum plasma level), $T_{max}$ (time to maximum plasma level), AUC or plasma half-life of the drug, or improved level of the drug in blood, liver or another tissue. Non-limiting examples of drugs whose pharmacokinetics can be improved by ritonavir are described in U.S. Patent No. 6,037,157. In general, the amount of ritonavir in the tablet is sufficient to boost the *in vivo* pharmacokinetics of the co-formulated drug(s).

In yet another embodiment of this aspect of the invention, the pre-tabletting material comprises a solid dispersion of ritonavir and another therapeutic agent which is metabolized by cytochrome P450 oxidase, such as CYP3A4. Examples of these therapeutic agents include, but are not limited to, immunomodulators (e.g., cyclosporine or FK-506), anti-cancer or chemotherapeutic agents (e.g., taxol or taxotere), antibiotics (e.g., clarithromycin, erythromycin, or tolithromycin), antivirals (e.g., indinavir, atazanavir, lopinavir, nelfinavir, saquinavir,

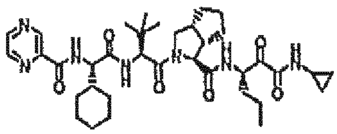

(compound VX-950, Vertex Pharmaceuticals Inc.),

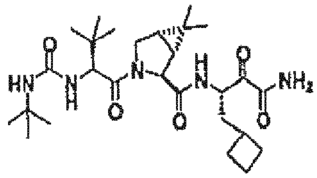

(compound SCH503034, Schering-Plough Co.),

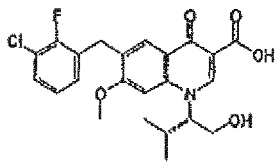

(compound GS9137, Gilead Sciences, Inc., Foster City, CA)), antihistamines (e.g., astemizole, chlorpheniramine, or terfenidine), calcium channel blockers (e.g., amlodipine, diltiazem, felodipine, lereanidipine, nifedipine, nisoldipine, nitrendipine, or verapamil), beta blockers (e.g., carvedilol, S-metoprolol, propafenone, or timolol), antidepressants (e.g., amitriptyline, clomipramine, desipramine, imipramine, or paroxetine), or prodrugs thereof.

In still another embodiment of this aspect of the invention, the pre-tabletting material comprises a solid dispersion of ritonavir and another therapeutic agent which is transported by P-glycoprotein. P-glycoprotein, also known as ATP-binding cassette sub-family B member 1, is an ATP-dependent efflux pump with broad substrate specificity. P-glycoprotein is expressed in a variety of cells such as those lining the intestine and those comprising the blood-tissue barriers. It is believed that P-glycoprotein functions as a membrane transporter and is capable of pumping out various drugs from cytoplasm, thereby reducing the bioavailability of these drugs. P-glycoprotein is also believed to be involved in multidrug resistance. Ritonavir can inhibit the activity of P-glycoprotein. Therefore, coadministration of ritonavir with a drug that is a substrate of P-glycoprotein can improve the absorption or bioavailability of the drug. Drugs that can be transported by P-glycoprotein include, but are not limited to, numerous HIV protease and reverse transcriptase inhibitors, chemotherapeutic agents, steroids, xenobiotic, and immunosuppressive agents.

The present invention also contemplates the use of a pre-tabletting material which comprises a solid dispersion of ritonavir and a solid dispersion of another therapeutic agent. The solid dispersion of ritonavir and the solid dispersion of the other therapeutic agent can be prepared separately and then combined to provide the pre-tabletting material. The solid dispersion of ritonavir and the solid dispersion of the other therapeutic agent can be prepared using the same polymer(s) and/or additives, or using different polymer(s) and/or additives.

In another aspect, the present invention features a pre-tabletting material which comprises a solid dispersion of at least one therapeutic agent in a matrix, where said at least one therapeutic agent comprises an HIV or HCV protease

inhibitor, and said matrix comprises one or more pharmaceutically acceptable hydrophilic polymers (or one or more pharmaceutically acceptable water-soluble polymers) and, optionally, one or more pharmaceutically acceptable surfactants. In one embodiment, the pre-tabletting material comprises from 5 to 30 % (preferably from 10 to 25 %) by weight of the total pre-tabletting material of an HIV or HCV protease inhibitor (or a combination of HIV or HCV protease inhibitors), at least 50% (preferably from 50 to 85 %, and more preferably from 60 to 80 %) by weight of the total pre-tabletting material of said one or more water-soluble/hydrophilic polymers, from 2 to 20 % (preferably from 3 to 15 %) by weight of the total pre-tabletting material of said one or more surfactants, and from 0 to 15 % by weight of the total pre-tabletting material of one or more other additives.

HIV protease Inhibitors suitable for use in the present invention include, but are not limited to, ritonavir, lopinavir, indinavir, saquinavir, 5 (S)- Boc- amino- 4 (S)- hydroxy- 6- phenyl- 2 (R) phenylmethylhexanoyl- (L)- Val- (L)- Phe-morpholin- 4- ylamide, 1- Naphthoxyacetyl- beta- methylthio- Ala- (2S, 3S) 3- amino- 2- hydroxy- 4- butanoyl- 1, 3-thiazolidine- 4t- butylamide, 5- isoquinolinoxyacetyl- beta- methylthio- Ala- (2S, 3S)- 3amino- 2- hydroxy- 4- butanoyl- 1, 3- thiazolidine- 4- tbutylamide, [1S- [1R- (R- ), 2S*] )- $N^1$ [3- [[[(1, 1- dimethylethyl) amino] carbonyl] (2- methylpropyl) amino]- 2hydroxy- 1- (phenylmethyl) propyl]- 2- [(2- quinolinylearbonyl) amino]- butanediamide, amprenavir (VX- 478), DMP- 323, DMP- 450, AG1343 (nelfinavir), atazanavir (BMS 232, 632), tipranavir, palinavir, TMC- 114, RO033- 4649, fosamprenavir (GW433908), P- 1946, BMS 186, 318, SC- 55389a, BILA 1096 BS, and U- 140690. HCV protease inhibitors suitable for use in the present invention include, but are not limited to, VX- 950, compound SCH503034, 1TMN-191/R7227, and TMC435350.

The tablets made according to the present invention preferably contain significantly less internal fractures, as compared to the tablets made from the same pre-tabletting material but using an unlubricated die and punches. More preferably, the tablets made according to the present invention do not contain any internal fracture that is detectable by XMT.

The present invention also features a tablet comprising a solid dispersion of at least one therapeutic agent in an above- described matrix, wherein the tablet does not contain any lubricants, or contains only an insignificant amount of lubricant (s), and wherein the tablet does not contain (1) any therapeutic agent that is denaturalized or inactivated when compressed at a pressure of greater than or equal to 1 ton/$cm^2$, (2) any low molecule active ingredient the elution of which is delayed when compressed at a pressure of greater than or equal to 1 ton/$cm^2$, or (3) any therapeutic agent that is affected by the lubricant (s) comprised in the tablet (if any) or any lubricant used on the die wall and/or compression surfaces. As used herein, an ingredient in a tablet is in an "insignificant" amount if the tablet having the ingredient has no detectable structural difference from an otherwise identically prepared tablet except that the latter tablet does not have the ingredient. Any therapeutic agent or agents described herein can be formulated in such a tablet.

In addition, the present invention features a tablet comprising a solid dispersion of at least one therapeutic agent in an above-described matrix, wherein the tablet does not contain any lubricants, or the total amount of lubricant (s) in the tablet is less than 0.5% by weight of the tablet, and wherein the tablet does not contain (1) any therapeutic agent that is denaturalized or inactivated when compressed at a pressure of greater than or equal to ton/$cm^2$, (2) any low molecule active ingredient whose elution is delayed when compressed at a pressure of greater than or equal to I ton/$cm^2$, or (3) any therapeutic agent that is affected by the lubricant(s) comprised in the tablet (if any) or any lubricant used on the die wall and/or compression surfaces. In some cases, the total amount of lubricant(s) in the tablet is less than 0.25% by weight of the tablet. Preferably, the total amount of lubricant(s) in the tablet is less than or 0.1%, 0.03%, 0.01% or lesser by weight of the tablet. Any therapeutic agent or agents described herein can be formulated in such a tablet.

In order to facilitate the intake of a tablet by a mammal, it is advantageous to give the tablet an appropriate shape. Large tablets that can be swallowed comfortably are therefore preferably elongated rather than round in shape.

A film coat on the tablet can further contribute to the ease with which it can be swallowed. A film coat can also improve taste and provide an elegant appearance. If desired, the film-coat may be an enteric coat. The film-coat usually includes a polymeric film-forming material such as hydroxypropyl methylcellulose, hydroxypropylcellulose, and/or acrylate or methacrylate copolymers, Besides a film-forming polymer, the film-coat may further comprise a plasticizer, e.g. polyethylene glycol, a surfactant, e.g. a Tween® type, and optionally a pigment, e.g. titanium dioxide or iron oxides. The film-coating may also comprise talc as anti-adhesive. The film coat usually accounts for less than 5 % by weight of the dosage form (unless the film coat is a control release coat or an active coat).

The exact dose and frequency of administration depends on the particular condition being treated, the age, weight and general physical condition of the particular patient as well as other medication the individual may be taking.

The presence of the lubricant layer(s) on a tablet can be detected using the Secondary Ion Mass Spectrometry (SIMS) or other suitable techniques. SIMS is the mass spectrometry of ionized particles which arc emitted from the surface when energetic primary particles bombard the surface. The pulsed primary ions with the energy of 1-25keV, typically liquid metal ions such as $Ga^+$, $Cs^+$ and $O^-$, can be used to bombard the sample surface, causing the secondary elemental or cluster ions to emit from the surface. The secondary ions are then electrostatically accelerated Into a field-free drift region. The ion with lower mass has higher flight velocity than one with higher mass. Thus they will reach the secondary-ion detector earlier. As a result, the mass separation is obtained in the flight time t from the sample to the

detector. A variety of mass ions are recorded by the detector with the time sequence to give the SIMS spectrum.

It should be understood that the above-described embodiments and the following examples are given by way of illustration, not limitation. Various changes and modifications within the scope of the present invention will become apparent to those skilled in the art from the present description.

**Example 1**

Compression of tablets containing a high percentage of polymeric excipients presents a significant challenge. As demonstrated below, polymers may have significant volume expansion after compaction, leading to the propagation of internal micro-cracks and large shear planes. Polymers can also exhibit high wall friction. Without limiting the present invention to any particular theory, it is postulated that the combination of these effects may lead to structural failure during tablet compression or upon storage.

When compared to normal excipients used in the manufacture of tablets, polymers exhibit significantly high elastic recovery, XMT images indicated that internal shear failure planes developed Inside the polymer-based tablet. Furthermore, with an increase in compression force, the failure planes became aligned perpendicular to the applied force. This realignment of shear failure planes may significantly produce failure in process. Moreover, particle size distribution can also significantly alter the formation of internal shear failure planes inside the tablet. Fine particles showed a reduction of shear failure planes compared to coarse particles.

A strong dependence between the internal failure planes and the processing variables like particle size distribution and compaction load is also demonstrated below. With an increase in applied force during compaction, the internal shear failure planes realign. This acts as a precursor to capping or delamination. The combination of compaction simulation and XMT provides an excellent tool to assess tendency towards failure in process.

Internal structural flaws often remain undetected by visual inspection. The flaws may result in capping during post compression processing. The present invention analyzed the internal structure of a tablet in a non-destructive manner. X-ray microtomography, capable of looking at the internal structure of the tablets, was used to understand the structural inequalities inside the tablets thereby enabling madification of the operating parameters to eliminate capping incidence during manufacturing. The equipment described hereinbelow has a resolution of 2 microns and can accommodate a sample size of up to 2 cm. An analysis that links the operating parameters in manufacturing (using a compaction simulator) to the internal structural flaws enables process optimization so that capping of the tablets can be minimized or prevented.

*Procedures*

Tablets were compressed on a Presster compaction simulator (Metropolitan Computing Corp., New Jersey, USA). A Skyscan X-ray micro-tomograph (model Skyscan 1172; SKYSCAN, Belgium) was used to generate the scans.

The Presster simulator is a linear compaction simulator that imitates the rotary compaction presses. A hopper holds the powder, which is discharged into the die just before engagement. The die and the punches move on a linear rail and the ramps located underneath the bottom punch raises the bottom punch during compaction and ejection. It has two wheels that engage on the top punch to create the necessary compressing forces. The wheels can be lowered or raised manually or electronically to increase or decrease the compaction force. By changing the linear velocity of the die table movement, the dwell time can be varied. This system is versatile in replicating the rotary presses. For the study described herein, the operating load for the pre-compaction was set at 9kN. The compaction force was varied from 36 to 42 to 48 kN.

The extrudate was prepared according to Examples 2 and 3 of U.S. Patent Application Publication No. 20050084529 except that Span 20 was added during extrusion, The entire content of U.S. Patent Application Publication No. 20050084529 is incorporated herein by reference. The compositions of the extrudate and the post-extrusion blend are described in Table 1.

Table 1. Compositions of Target Milled Extrudate and Post-Extrusion Blend

| Ingredients | Parts by Weight |
|---|---|
| ***Target Milled Extrudate*** | |
| Lopinavir | 200 |
| Ritonavir | 50 |
| Copovidone K28 (Kollidon VA 64; BASF) | 853.8 |
| Sorbitan monolaurate | 83.9 |

(continued)

| Ingredients | Parts by Weight |
|---|---|
| ***Target Milled Extrudate*** | |
| Colloidal silicon dioxide | 12.0 |
| ***Additional Ingredients in Post-Extrusion Blend*** | |
| Sodium stearyl fumarate | 12.3 |
| Colloidal silicon dioxide | 8.0 |

The extrudate were milled to the desired particle size distribution, The particle size distribution of the milled extrudate (hereinafter the target milled extrudate) is shown in Figure 2A. The fine extrudate and the coarse extrudate were separated by sieving the target milled extrudate using 170 micron mesh (the upper cut being the coarse), and their particle size distributions are depicted in Figure 2B and 2C, respectively.

Apart from the milled extrudate, Avicel® PH101 (microcrystalline cellulose; FMC BioPolymer, Philadelphia, PA) and fast flow lactose were used for the analysis. Avicel® PH101 has been historically considered to be a material that is easy to compress due to plasticity, whereas pure lactose is brittle and Is very challenging to tabletting process.

***On-line Lubrication System***

The on-line lubrication unit Fette PKB 2 (PETTE GMBH, Hamburg, Germany) was used to lubricate die wall. The system includes a loss-in-weight feeder enabling for constant flow of the lubricant(s) (e.g., sodium stearyl fumarate), a slit nozzle to atomize and spray the lubricant(s) onto the surfaces of the press chamber prior to filling of the die with the pre-tabletting powder, and a suction unit to remove excess lubricant(s) from rotor and die to avoid uncontrollable dust exposure during the compression process. The material flow from feeder to nozzle was conducted or controlled by pressurized air. Both components were linked by flexible tubing.

The spray rate can be adjusted, and the actual value was displayed. For common compression processes the feeding rate was in the range of 100-800 grams per hour, depending on the characteristics of the to-be-compressed powder blend and other factors. Start and stop of the feeding cycle can be controlled manually or, if connected to a Fette control terminal, automatically in-line with the start/stop signal of the tablet press. Charging of the lubricant container which holds up to 3 kg of lubricant is a manual process.

***Presster Compaction Simulations***

During the compaction simulation, one significant observation was the high elastic recovery of the tablets. This was observed from the force versus displacement graph for the material at various compaction forces for the lubricated blend (the target milled extrudate blended with sodium stearyl fumarate and colloidal silicon dioxide, as showed In Table 1) and the unlubricated blend. Various reasons may be attributed to this behavior. The application of high compaction force may be one factor that can contribute to this observation as the material could have passed through its maximum plastic deformation. When the material is over compressed, the tendency for the top punch to bounce back might also be the reason for the high value of elastic recovery. The design of the punches could also contribute to the high elastic recovery. In addition, it was observed that the elastic recoveries arc different between the lubricated and unlubricated blends, whereas there was no significant difference between the elastic recoveries of the over lubricated (i.e., blended with the same amount of lubricant(s) as in the lubricated blend but the blending process took an extended poriod of time) and the lubricated blends. For the unlubricated tablets, an elastic recovery of 14.2 was observed whereas for Avicel and lactose, these values were 7 and 6.4, respectively. This observation of high elastic recovery of the unlubricated blend was an indication of propensity to cap in process.

Using the density of the material and the thickness changes during the compaction, the Hockel plots were constructed. One observation from the Heckel plot was the lack of major deformation in the initial stages of compaction for the milled extrudate. The initial variation in the slope, which is attributed to this rearrangement, was mostly absent in the polymer-containing formulations that were tested. This might be due to either large deformation of individual particles attributed to elasticity or due to high inter-particle friction that prevents slip between particles. Even at this high compression, fast flow lactose and Avicel® PH101 showed these deformations.

The high elasticity of the material was reconfirmed from the Heckel plot. The deviation from the horizontal for different materials is an indication of this elasticity. Even Avicel® PH101 showed this high deformation and hence it can be concluded that the operating loads need to be lowered to avoid this high elasticity.

From the inverse of the slope of initial deformation part of the Heckel plot, the mean yield pressure was obtained for different materials (Figure 3). The unlubricated blend when compressed with die wall lubricated ("Unlubed Meltrex with die wall tubing") appeared to be better than the lubricated blend without die wall lubrication ("Target blend") and even Avicel® PH101 in terms of compressibility. The unlubricated blend without die wall lubrication ("Unlubed Meltrex") was worse than fast flow lactose with die wall lubrication ("Lactose with wall lubing").

The above analysis demonstrated the presence of large elastic deformation towards the capping of polymer-based tablets.

### *X-Ray Microtomography*

X- ray images of the tablets made from the lubricated blend without die wall lubrication showed significantly deep internal cracks extending into the material matrix. The tablets showed internal failure planes extending from the side of the tablet. This means that the tablets had structurally failed even though optically they appear to be perfect. A large number of micro cracks were also observed for these materials. It appears that the large cracks have a tendency to form first and then orient perpendicular to the applied force with increasing applied compaction force. The micro cracks appear to undergo a reorientation inside the material matrix during ejection.

Figures 4A, 4B and 4C illustrate the X-ray images of exemplary tablets made of the target milled extrudate and compressed in an unlubricated die at 36 kN, 42 kN and 48 kN, respectively. Figures 4D and 4E show the X-ray images of representative tablets made of the target milled extrudate and compressed in a lubricated die at 42 kN and 48 kN, respectively.

### *Effect of Particle Size Distribution*

It was observed that particle size distribution has a significant impact on tablet internal structure. XMT images showed that tablets compacted from the fine extrudate at 36 kN had significantly less internal fractures and micro cracks and, in many instances, did not have any detectable internal fractures or micro cracks (Figure 5A without die wall lubrication; Figure 5B with die wall lubrication). The Heckel plot during compaction showed that the fine extrudate underwent reorganization in the initial stages of compaction, whereas the target milled extrudate and the coarse extrudate did not show this behavior. This reorganization of particles during the initial phase of compaction may explain the significantly reduced internal structural failure of the fine extrudate. Another significant observation was that the XMT images between the fine extrudate compressed in unlubricated die (Figure 5A) and the fine extrudate compressed in lubricated die (Figure 5B) did not show significant difference in internal fractures. For the fine extrudate, the shear failure planes started to appear at 42 kN main compression force without die wall lubrication (Figure 6A; compared to Figure 6B with die wall lubrication) and increased in depth when the main compression force was increased to 48 kN (Figure 7A without die wall lubrication Figure 7B with die wall lubrication).

Shear failure planes were also observed for tablets made of coarse particles without die wall lubrication. For instance, Figure 8A shows an exemplary tablet made of coarse particles and compressed in an unlubricated die at 42 kN. A large amount of micro cracks were observed for these course particles. This indicated that particle size distribution has a significant influeince on how the compact behaves during compression.

### *Effect of Die Wall Lubrication*

As demonstrated in Figures 4B vs. 4D, 4C vs. 4E, 6A vs. 6B, 7A vs. 7B, and 8A vs. 8B, tables compacted with lubricated die wall (Figures 4D, 4E, 6B, 7B, and 8B) showed significantly less internal fractures or micro cracks than tablets compacted without die wall lubrication (Figures 4B, 4C, 6A, 7A, and 8A, respectively). The effect of die wall lubrication on internal fractures was observed for both the fine extrudate and the coarse extrudate.

Figure 9 depicts the XMT images of a representative tablet made of the coarse extrudate and compressed at 48 kN with die wall lubrication, demonstrating a significantly improved internal structure.

### *Conclusions*

Without limiting the present invention to any particular theory, it is believed that polymers have relatively higher friction with stainless steel and tend to expand after compaction. The elastic recovery of the tablet can lead to internal microcracks due to polymer expansion above hydrostatic state. Moreover, due to the wall friction, shear failure zones are developed, separating the free and sheared surfaces and leading to capping.

The compaction of the target milled, fine and coarse extrudates were studied using an MCC Presster compaction simulator and X-ray microtomography. It was observed that the target milled extrudate has a higher elastic recovery than traditional Avicel-Lactose based tablets. The flexibility of the material led to large, shear failure zones that created

tablet failure.

The mean yield pressure values obtained by the Heckel analysis indicated that the target milled extrudate was challenging to compress without the lubricant. When the wall friction was reduced using die wall lubrication, compression was facilitated. X-ray microtomography was also used to image the inside of the tablets nondestructively and it was observed that tablets made with the coarse extrudate and the target milled extrudate showed a tendency to develop micro cracks inside the tablet core. These cracks can grow and develop Into failure planes. Moreover, it was observed that the cracks underwent a reorientation with increased compaction load. They tended to align perpendicular to the normal applied load at high compaction loads. This observation provides an explanation to material failure in process and can be used as a tool for process optimization. This tendency was observed for tablets that showed capping during demonstration runs.

In all tested cases the shear failure planes disappeared or significantly reduced when die wall lubrication was used. Hence, by lubricating die walls, the structural integrity of the tablets can be improved. The use of the die wall lubrication can lead to processability improvement, thereby eliminating or reducing capping ore delamination in process. Particle size distribution also showed an important role in the formation of micro cracks. The use of more fine particles during compaction can therefore significantly improve the structural integrity of the tablets during processing.

**Example 2**

A lubricant feed rate - ejection force profile, and subsequently a compression force - hardness profile at a constant lubricant feed rate, were recorded. Compared to the process without die wall lubrication, the tablet hardness specification could be met at lower compression forces. The tolerability of the ejection force to slight variations of the lubricant feed rate was acceptable with respect to consistent ejection forces, showing the robustness of the process.

Lubricant was removed from the pre-tabletting powder blend, and the surfaces of the press chamber were layered with lubricant. These were achieved by using a lubricant spraying system (Fette PKB 2) which provided a constant flow of magnesium stearate, sodium stearyl fumarate or other suitable lubricants. The lubricant was sprayed into the press chamber by means of pressurized air. Afterwards excess material was removed and the press chamber was filled with non-lubriated pre-tabletting powder for compression.

The milled extrudate was prepared as described in Example 1 and comprised 16.67% lopinavir, 4.17% ritonavir, 71.16% copovidone K28, 7% Span 20, and 1% silicon dioxide. The milled extrudate was compressed, and no precursory blending with additives like lubricants or glidants was performed. For every extrudate a lubricant feed rate - ejection force profile, as well as a compression force - hardness profile at an appropriate feed rate, were recorded. The lubricant used was sodium stearyl fumarate. Compression runs were conducted on a Fette 1200 (FETTE GMBH, Hamburg, Germany), which was coupled with Fette PKB 2 to provide on-line lubrication of die wall. Main compression force was varied from 14.8 to 41.9 kN. During compression, rotary speed, fill-o-matic speed, main compression force, ejection force and lubricant feed rate were recorded. Front all trials a sample was taken and tested for tablet hardness and friability.

With die wall lubrication, the milled extrudate can be easily compressed with compression forces of 21 kN or above. When the milled extrudate was blended with 1% sodium stearyl fumarate and 0.66% silicon dioxide, the blend had to be compressed (without die wall lubrication) at 30-40 kN to achieve a tablet hardness in a comparable range. It was also observed that sodium stearyl fumarate concentrations from 0.05% to 0.3% had a significant impact on tablet hardness if blended with the extrudates prior to compression but without die wall lubrication. With on-line die wall lubrication, tablet hardness was not affected as the core of the tablet is nearly lubricant-free. The ejection forces seemed to be quite constant in a relatively small range with respect to the lubricant feed rate.

The on-line lubrication process may offer several advantages compared to the processes where milled extrudates are blended with lubricants and then compressed in unlubricated die. These advantages include significantly shorter process flow (e.g., blending for lubricant can be removed), no scaling-up of blending process, reduced number of process parameters and variables, facilitating a continuous manufacturing process, and enabling for pure tablet cores without influence of compression additives.

The foregoing description of the present invention provides illustration and description, but is not intended to be exhaustive or to limit the invention to the precise one disclosed. Modifications and variations are possible in light of the above teachings or may be acquired from practice of the invention. Thus, it is noted that the scope of the invention is defined by the claims and their equivalents.

In addition, the present Invention further relates to the following embodiments:

1. A process of making tablets, comprising:

compressing a pre-tabletting material in a die to form a tablet, wherein an Internal surface of the die is lubricated with at least one lubricant, and said pro-tabletting material comprises at least one therapeutic agent and at least one pharmaceutically acceptable polymer; and

ejecting said tablet from said die,
wherein said pre-tabletting material does not include (1) any therapeutic agent that is denaturalized or inactivated when compressed at a pressure of greater than or equal to I ton/$cm^2$, (2) any low molecule active ingredient the elution of which is delayed when compressed at a pressure of greater than or equal to 1 ton/$cm^2$, or (3) any therapeutic agent that is affected by said at least one lubricant.

2. The process according to 1, wherein said pre-tabletting material comprises a solid dispersion of said therapeutic agent in a matrix, and said matrix comprises said pharmaceutically acceptable polymer.

3. The process according to 2, wherein said pre-tabletting material comprises at least 30% by weight of said at least one pharmaceutically acceptable polymer.

4. The process according to 3, wherein said matrix further comprises at least one pharmaceutically acceptable surfactant.

5. The process according to 4, wherein said pharmaceutically acceptable polymer is a homopolymer or copolymer of N-vinyl pyrrolidone, and said pharmaceutically acceptable surfactant has an HLB value of from 4 to 10.

6. The process according to 5, wherein said pharmaceutically acceptable polymer is copovidone, and said pharmaceutically acceptable surfactant is sorbitan monolaurate.

7. The process according to 6, wherein said therapeutic agent is ritonavir.

8. The process according to 7, wherein said pre-tabletting material further comprises lopinavir.

9. The process according to 7, wherein said at least one lubricant comprises sodium stearyl fumarate.

10. The process according to 9, wherein said pre-tabletting material is compressed In the die between a lower surface of an upper punch and an upper surface of a lower punch, and said lower surface and said upper surface are lubricated with said at least one lubricant.

11. The process according to 7, wherein said tablet shows a dose-adjusted AUC of ritonavir plasma concentration in dogs, under non-fasting conditions, of at least 5 μg·h/ml/100 mg.

12. The process according to 8, wherein said tablet shows a dose-adjusted AUC of ritonavir plasma concentration in dogs, under non-fasting conditions, of at least 5 μg·h/ml/100 mg, and a dose-adjusted AUC of lopinavir plasma concentration in dogs, under non-fasting conditions, of at least 15 μg·h/ml/100 mg.

13. The process according to 3, wherein said pre-tabletting material is prepared by melt extrusion which comprises the steps of
solidifying a melt comprising said at least one therapeutic agent and said at least one pharmaceutically acceptable polymer; and
milling said solidified melt to provide said pre-tabletting material.

14. The process according to 3, wherein said at least one therapeutic agent comprises an anti-cancer or anti-pain agent.

15. A process of making tablets, comprising:

compressing a granular or powdery pre-tabletting material In a die to form a tablet,
wherein said granular or powdery material comprises at least one therapeutic agent and at least one pharmaceutically acceptable polymer, and at least 90% of particles In said granular or powdery material are smaller than 400 μm; and
ejecting said tablet from said die.

16. The process according to 15, wherein said pre-tabletting material comprises a solid dispersion of said at least one therapeutic agent in a matrix, and said matrix comprises said at least one pharmaceutically acceptable polymer.

17. The process according to 16, wherein said at least one therapeutic agent comprises ritonavir, or a combination of ritonavir and lopinavir.

18. A tablet comprising a solid dispersion of at least one therapeutic agent in a matrix, wherein said matrix comprises at least one pharmaceutically acceptable hydrophilic polymer and optionally, at least one pharmaceutically acceptable surfactant, wherein said tablet does not contain any lubricant, or the total amount of lubricant or lubricants in said tablet is less than 0.5% by weight of said tablet, and wherein said tablet does not contain (1) any therapeutic agent that is denaturalized or inactivated when compressed at a pressure of greater than or equal to 1 ton/$cm^2$, (2) any low molecule active ingredient the elution of which is delayed when compressed at a pressure of greater than or equal to 1 ton/$cm^2$, or (3) any therapeutic agent that is affected by said lubricant or lubricants.

19. A tablet comprising a compressed core which includes a solid dispersion of at least one therapeutic agent in a matrix, wherein said matrix comprises at least one pharmaceutically acceptable hydrophilic polymer and optionally, at least one pharmaceutically acceptable surfactant, wherein said core does not contain any lubricant therein, and said tablet does not contain (1) any therapeutic agent that is denaturalized or inactivated when compressed at a pressure of greater than or equal to 1 ton/$cm^2$, (2) any low molecule active ingredient the elution of which is delayed when compressed at a pressure of greater than or equal to 1 ton/$cm^2$, or (3) any therapeutic agent that is effected by any lubricant comprised in said tablet.

20. A pre-tabletting material comprising at least one therapeutic agent and at least one pharmaceutically acceptable polymer; wherein said pre-tabletting material does not contain any lubricant, or the total amount of lubricant or lubricants in said pre-tabletting material is less than 0.5% by weight of said pre-tabletting material, and wherein said pre-tabletting material does not include (1) any therapeutic agent that is denaturalized or inactivated when compressed at a pressure of greater than or equal to 1 ton/$cm^2$, (2) any low molecule active ingredient the elution of which is delayed when compressed at a pressure of greater than or equal to 1 ton/$cm^2$, or (3) any therapeutic agent that is affected by said at least one lubricant.

## Claims

**1.** A tablet made by a process comprising:

compressing a pre-tabletting material against at least one compression surface of a die at a pressure of less than 30kN/$cm^2$, wherein the compression surface is lubricated with at least one lubricant, and wherein the pre-tabletting material comprises

(1) less than 0.2% by weight lubricant,
(2) at least 30% by weight of at least one pharmaceutically acceptable polymer, and
(3) at least one therapeutic agent, wherein

a. the therapeutic agent is not denaturalized or inactivated when compressed at a pressure of greater than or equal to 10kN/$cm^2$,
b. the elution of the therapeutic agent is not delayed when compressed at a pressure of greater than or equal to 10kN/$cm^2$, and
c. the therapeutic agent is not affected by the lubricant.

**2.** The tablet of claim 1, wherein the lubricant comprises sodium stearyl fumarate.

**3.** The tablet of any one of claims 1-2, wherein the amount of the at least one lubricant in the tablet is less than 0.1% by weight of the tablet.

**4.** The tablet of any one of claims 1-3, wherein the polymer is water-soluble and has a $T_g$ of at least 50°C.

**5.** The tablet of any one of claims 1-4, wherein the polymer comprises a homopolymer or copolymer ofN-vinyl pyrrolidone.

**6.** The tablet of any one of claims 1-5, wherein the polymer comprises copovidone.

**7.** The tablet of any one of claims 1-5, wherein the therapeutic agent is present in the pre-tabletting material as a solid dispersion in a matrix comprising the polymer.

**8.** The tablet of claim 7, wherein compressing the pre-tabletting material further comprises compressing the pre-tabletting material into a compressed core, and wherein the compressed core includes the solid dispersion of the therapeutic agent in the matrix comprising the polymer.

**9.** The tablet of claim 8, wherein the core is coated with a film coat.

**10.** The tablet of any one of claims 1-9, wherein the pre-tabletting material further comprises at least one pharmaceutically acceptable surfactant.

**11.** The tablet of claim 10, wherein the surfactant has an HLB value of from 4 to 10.

**12.** The tablet of claim 11, wherein the surfactant comprises a sorbitan mono fatty acid ester.

**13.** The tablet of any one of claims 10-12, wherein the lubricant comprises sodium stearyl fumarate, wherein the polymer is copovidone, and wherein the surfactant is sorbitan monolaurate.

**14.** The tablet of any one of claims 1-15, wherein the compression surface of the die includes a lower surface of an upper punch and an upper surface of a lower punch, and wherein the lower surface and the upper surface are lubricated with the lubricant.

**15.** The tablet of any one of claims 1-13, wherein the therapeutic agent comprises ritonavir.

**16.** The tablet of claim 16, wherein the tablet shows a dose-adjusted AUC of ritonavir plasma concentration in dogs, under non-fasting conditions, of at least 5 $\mu$g·h/ml/100 mg.

**17.** The tablet of claim 16, wherein the therapeutic agent further comprises lopinavir.

**18.** The tablet of claim 18, wherein said tablet shows a dose-adjusted AUC of ritonavir plasma concentration in dogs, under non-fasting conditions, of at least 5 $\mu$g·h/ml/100 mg, and a dose-adjusted AUC of lopinavir plasma concentration in dogs, under non-fasting conditions, of at least 15 $\mu$g·h/ml/100 mg.

**19.** The tablet of any one of claims 1-18, wherein the process further comprises preparing at least a portion of the pre-tabletting material by melt extrusion.

**20.** The tablet of claim 19, wherein the melt extrusion comprises
solidifying a melt comprising therapeutic agent, polymer, surfactant and combinations thereof to form a solidified melt; and
reducing the solidified melt to granules.

**21.** The tablet of any one of claims 1-20, wherein at least 90% of particles in the pre-tabletting material have a mean particle size of less than 400 $\mu$m.

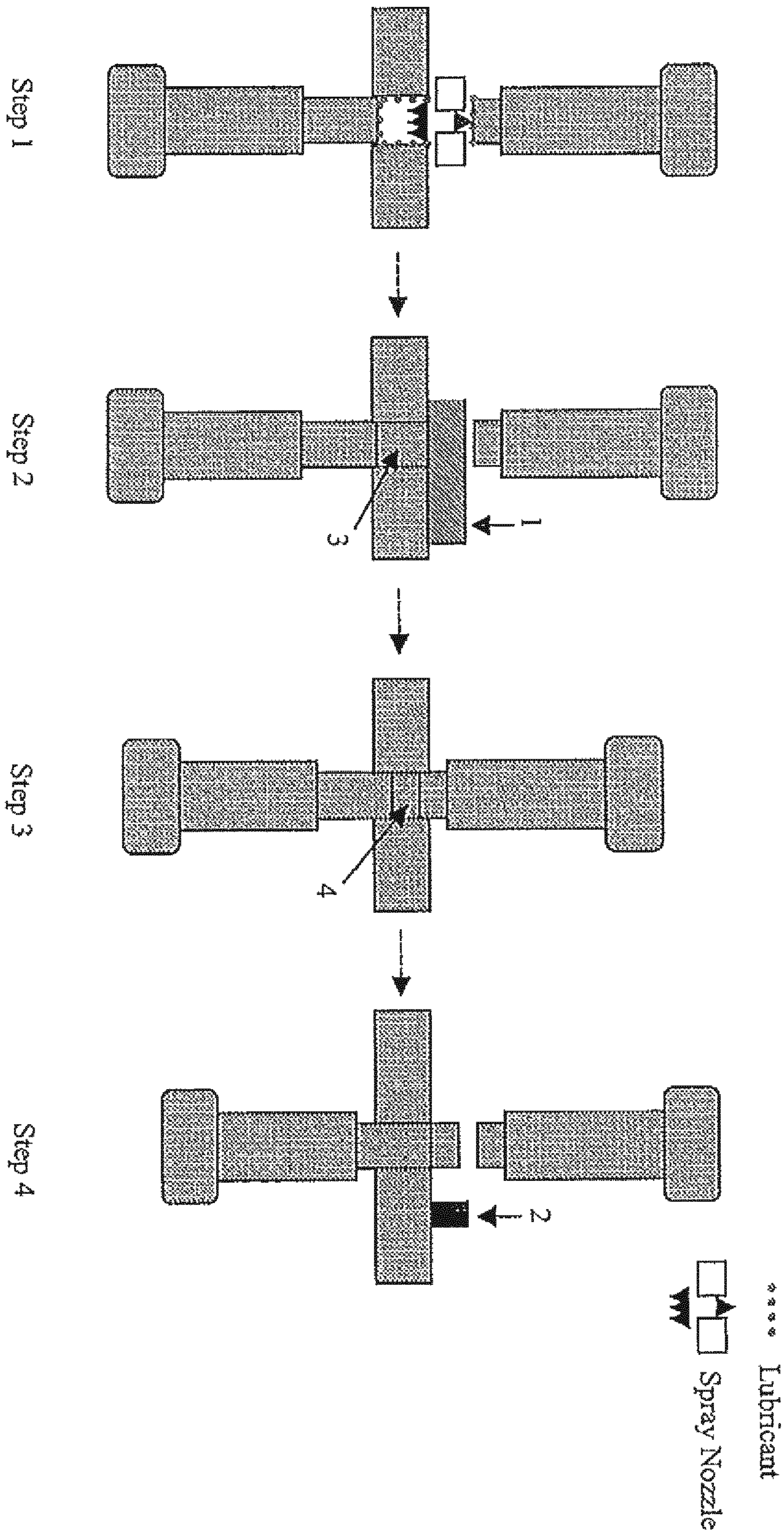


Figure 1

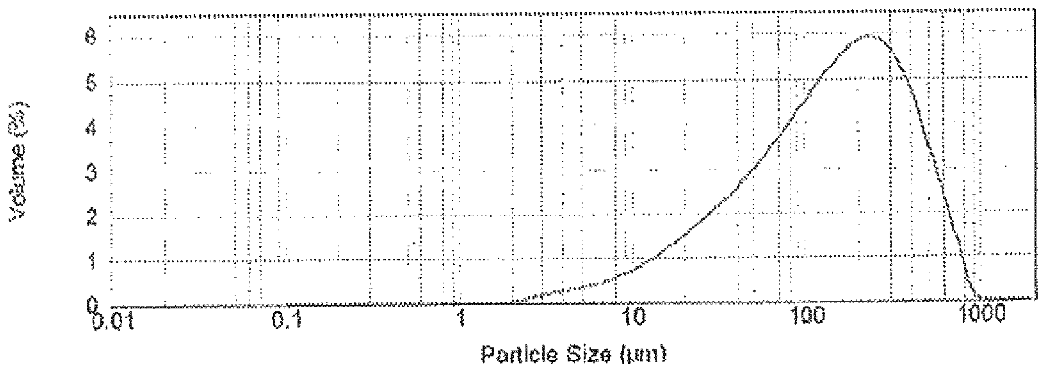


Figure 2A

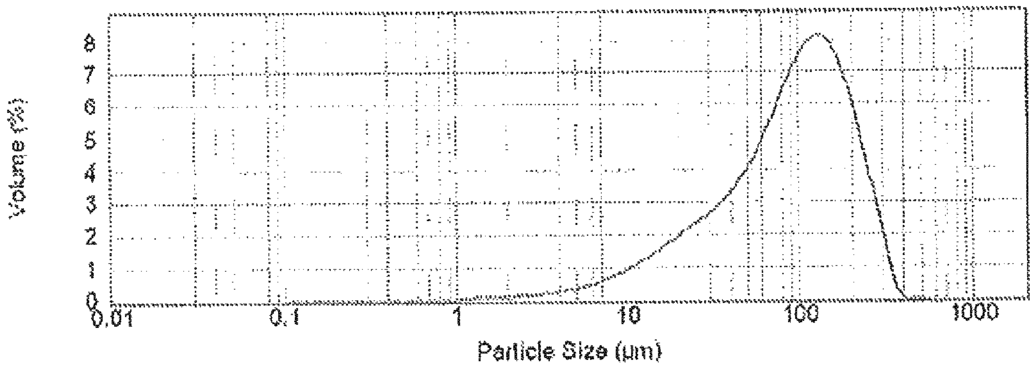


Figure 2B

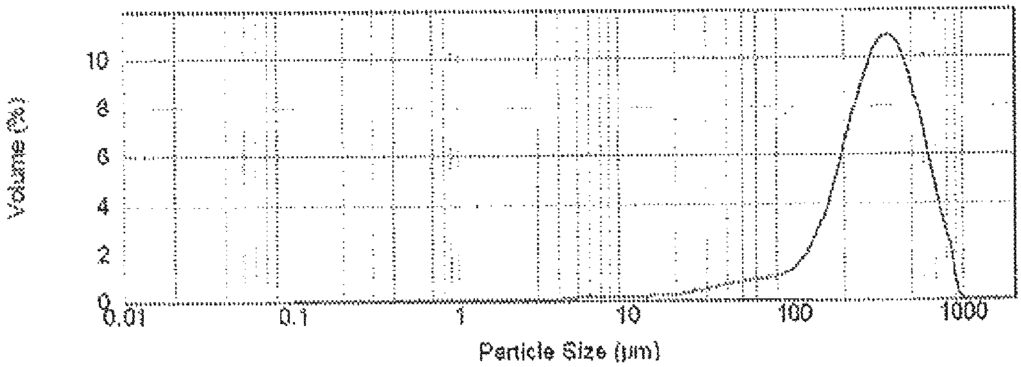


Figure 2C

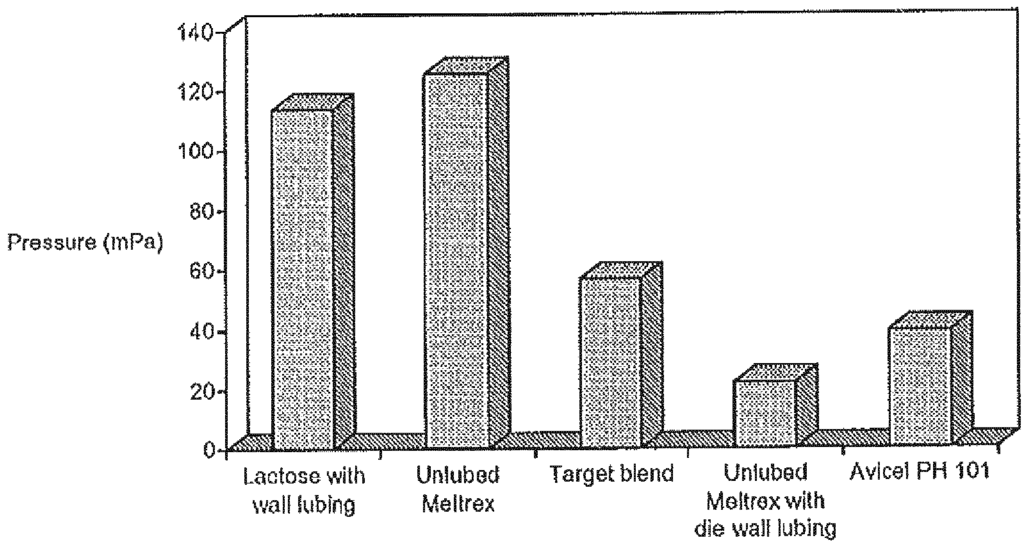
140
120
100
80
60
40
20
0
Pressure (mPa)
Lactose with wall lubing
Unlubed Meltrex
Target blend
Unlubed Meltrex with die wall lubing
Avicel PH 101

Figure 3

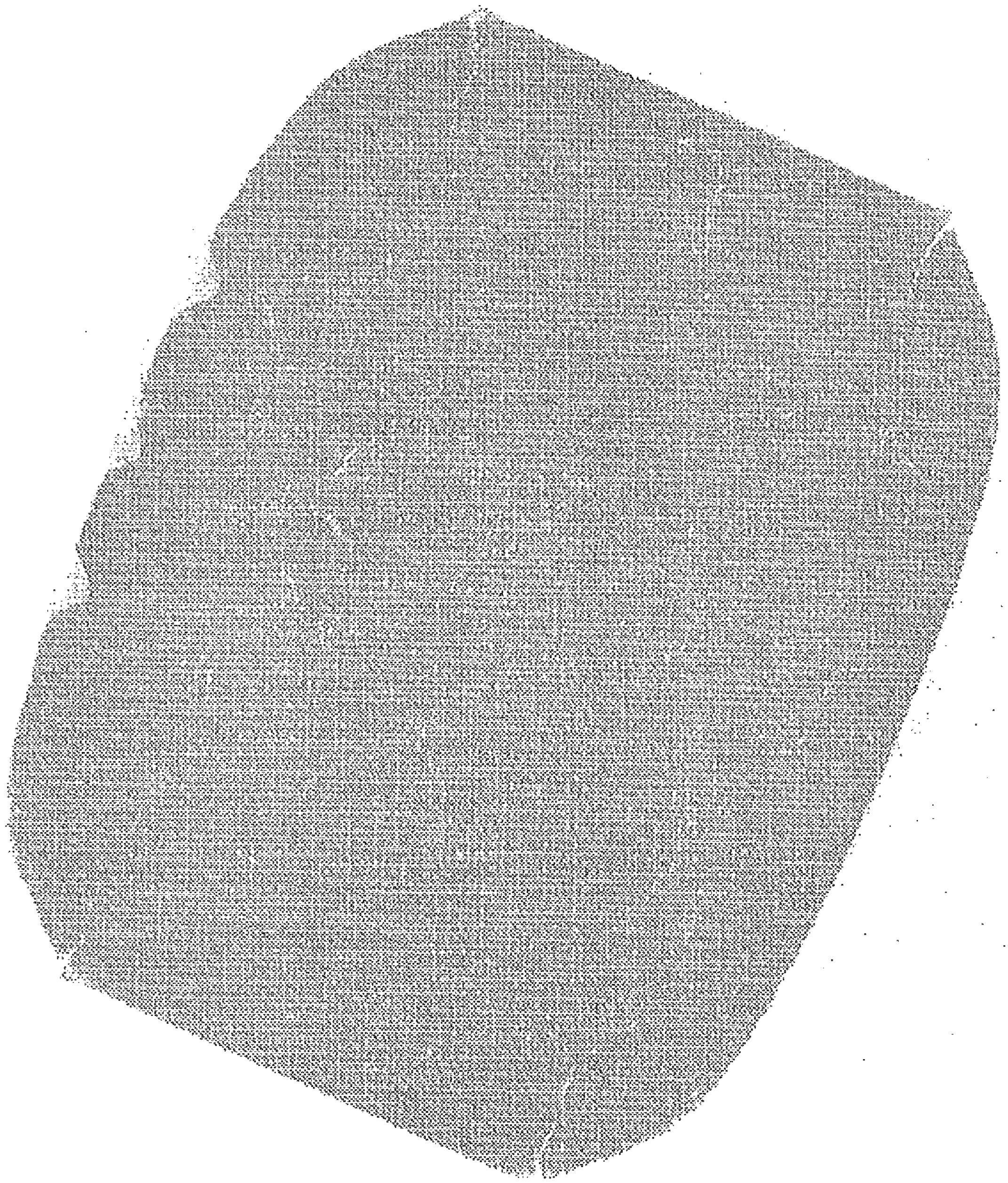

Figure 4A

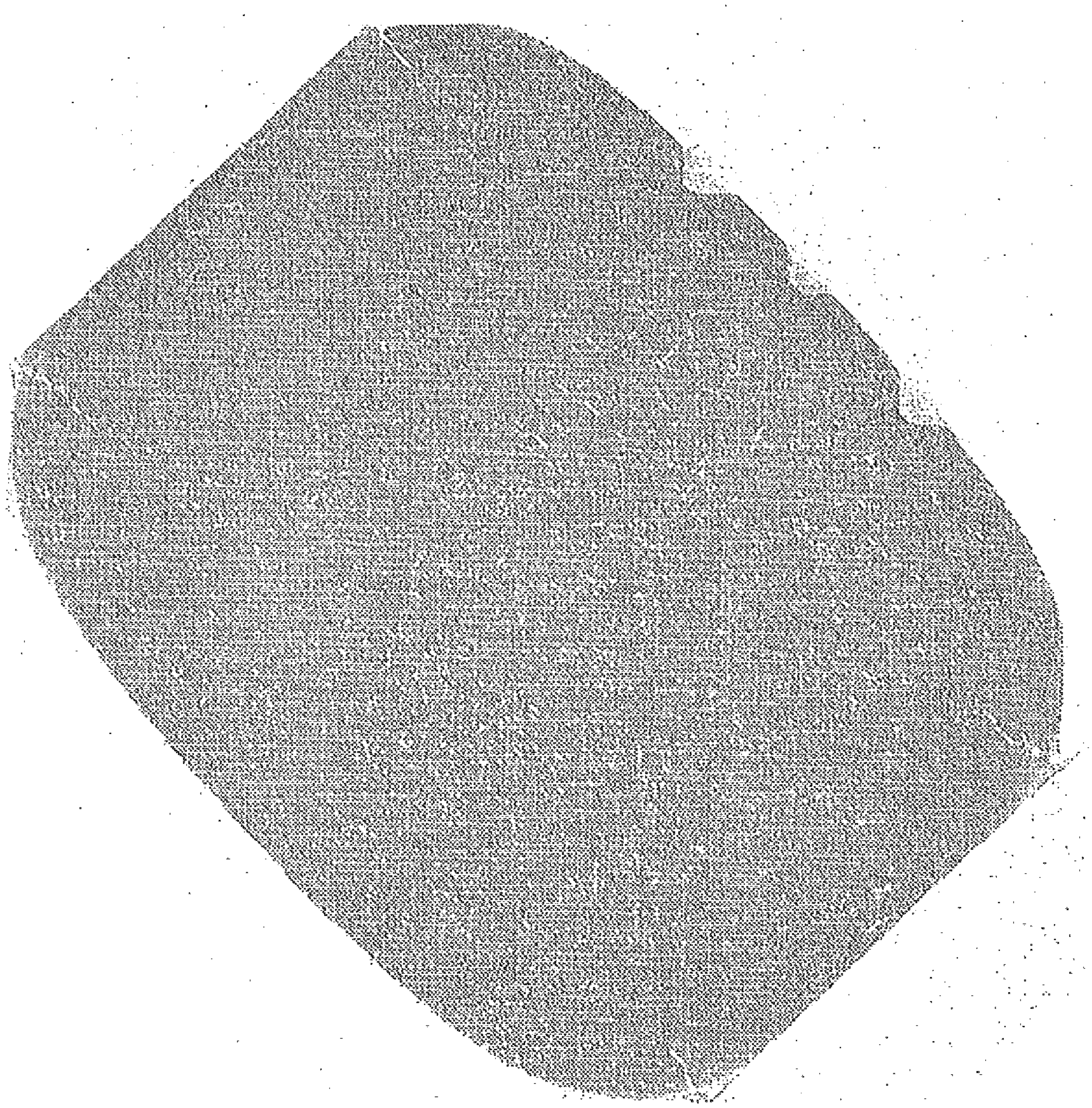

Figure 4B

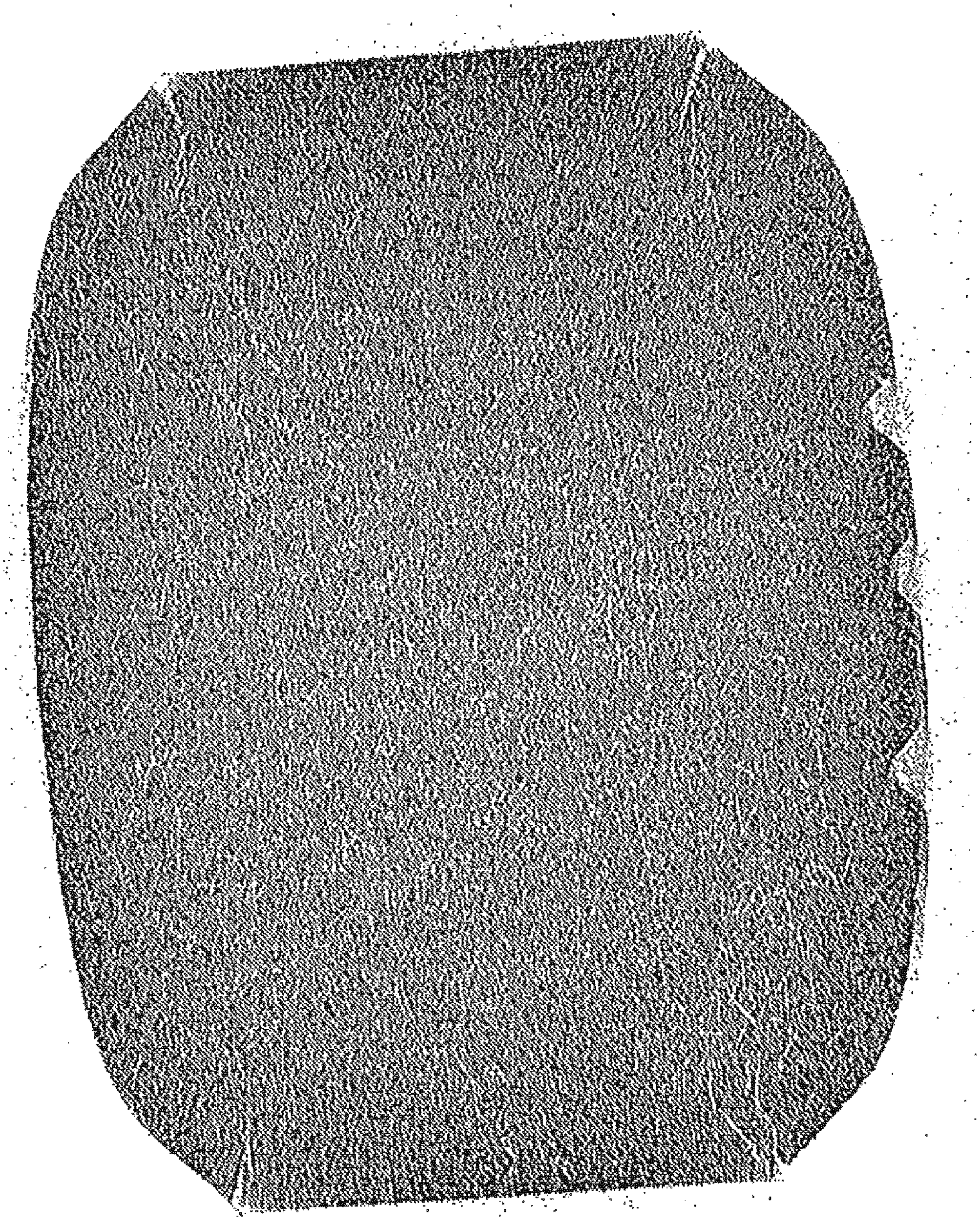

Figure 4C

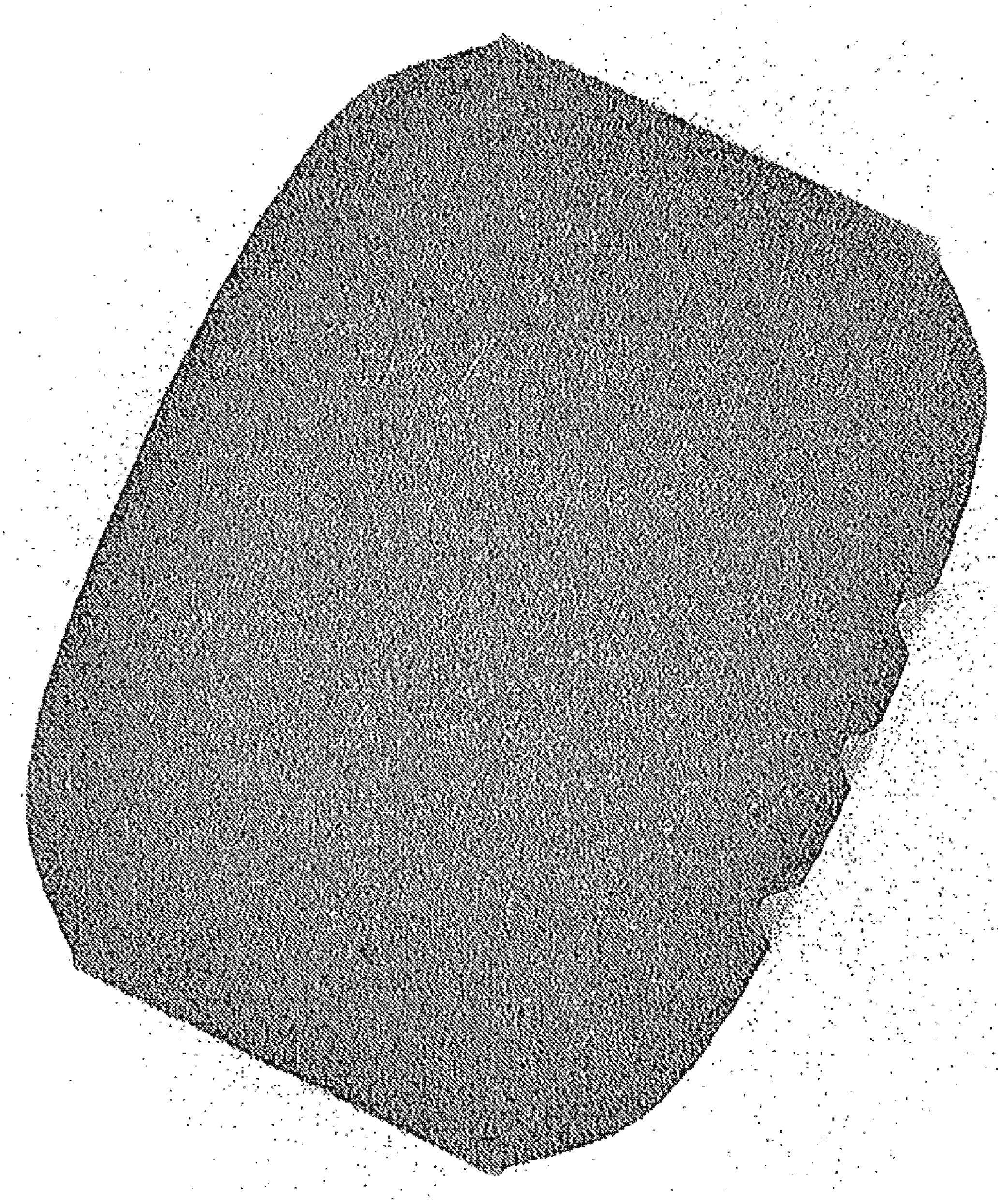

Figure 4D

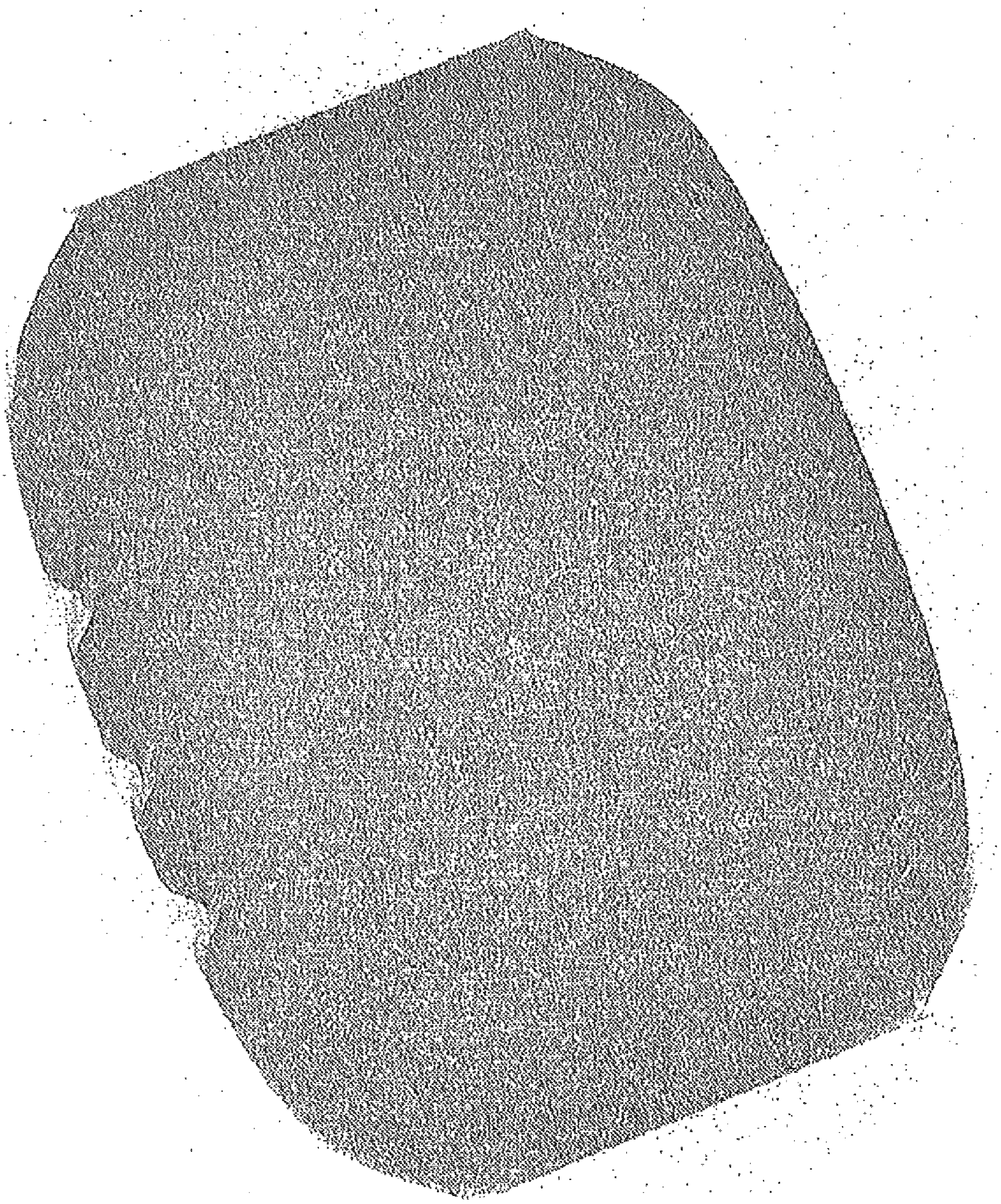

Figure 4E

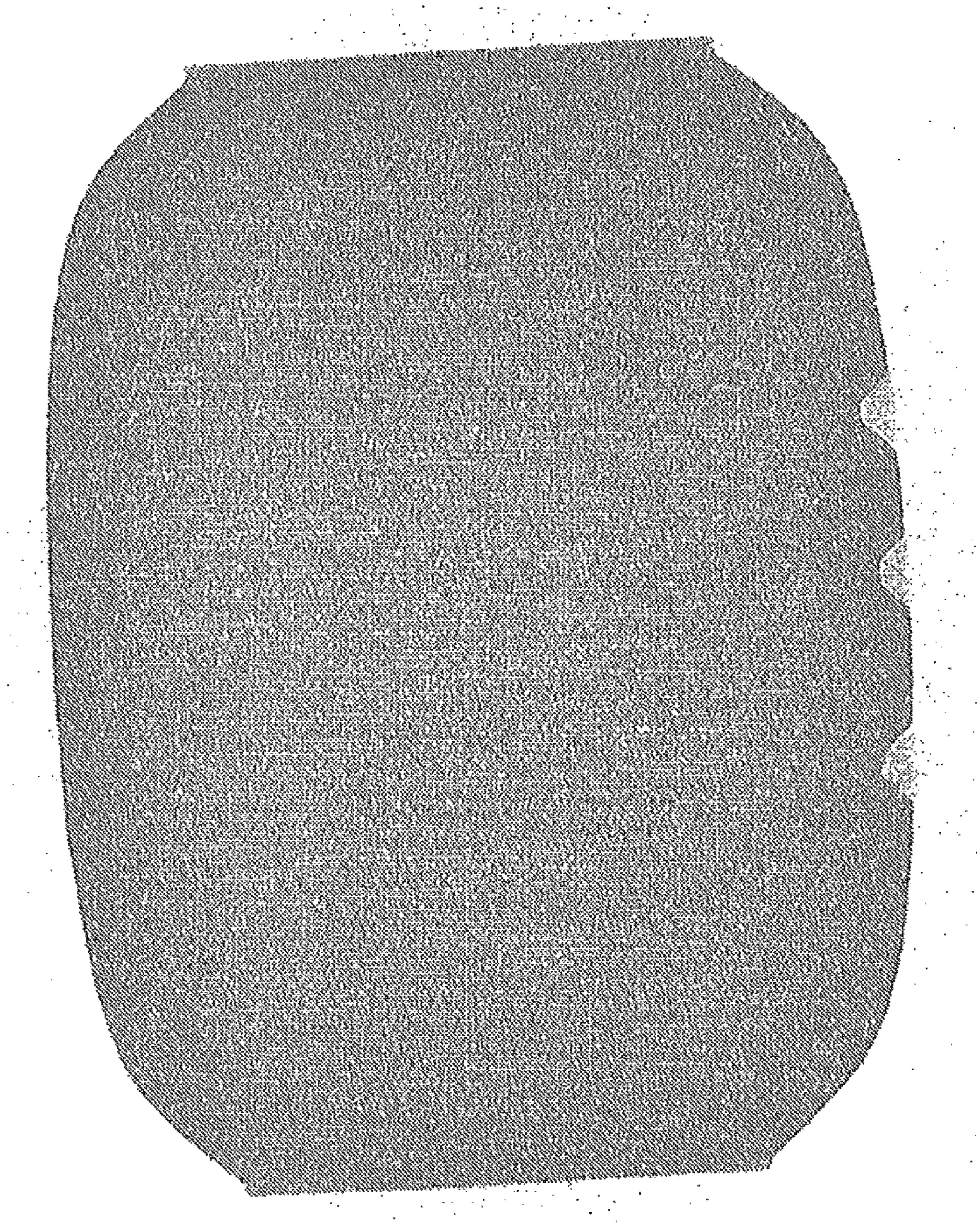

Figure 5A

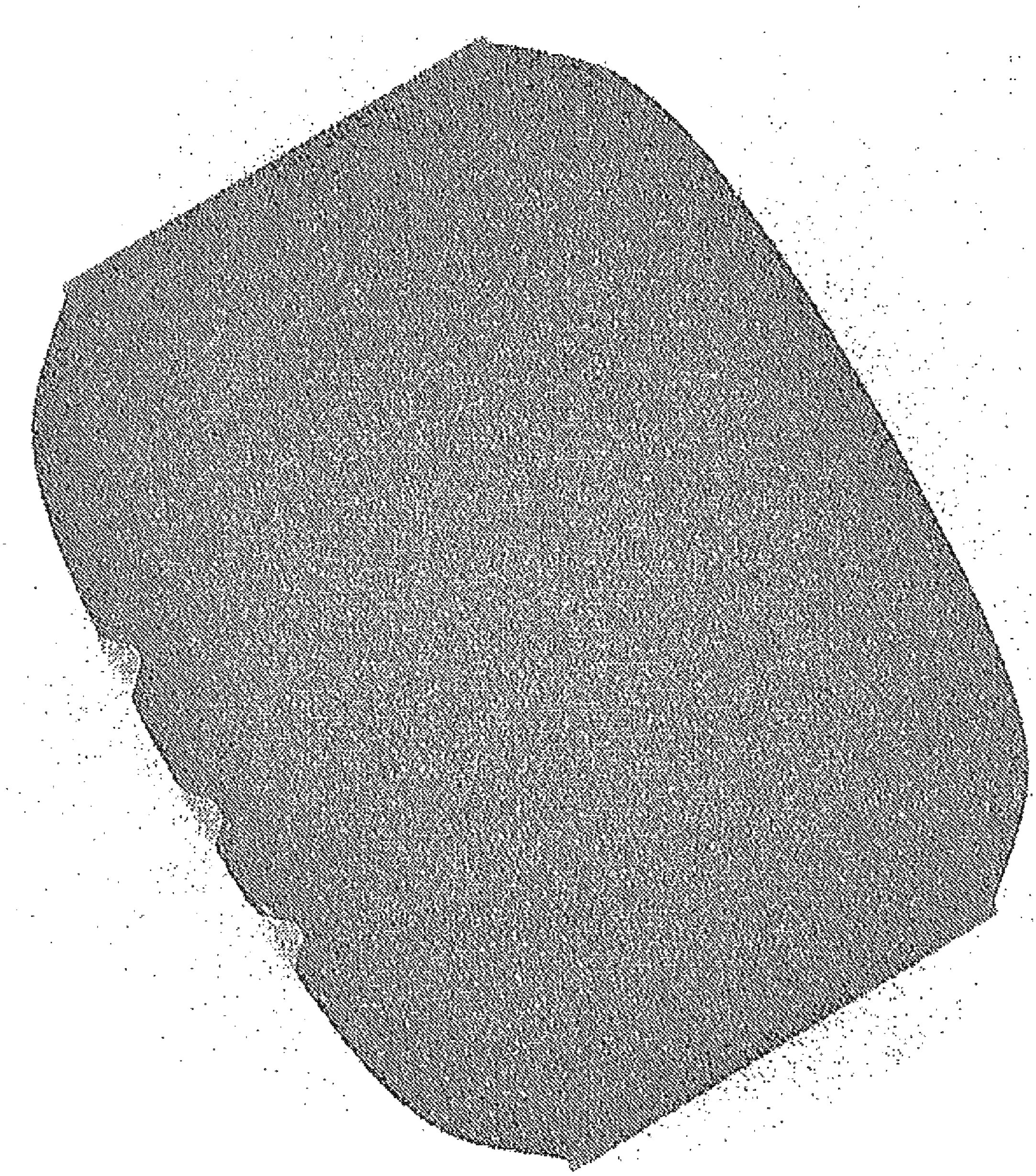

Figure 5B

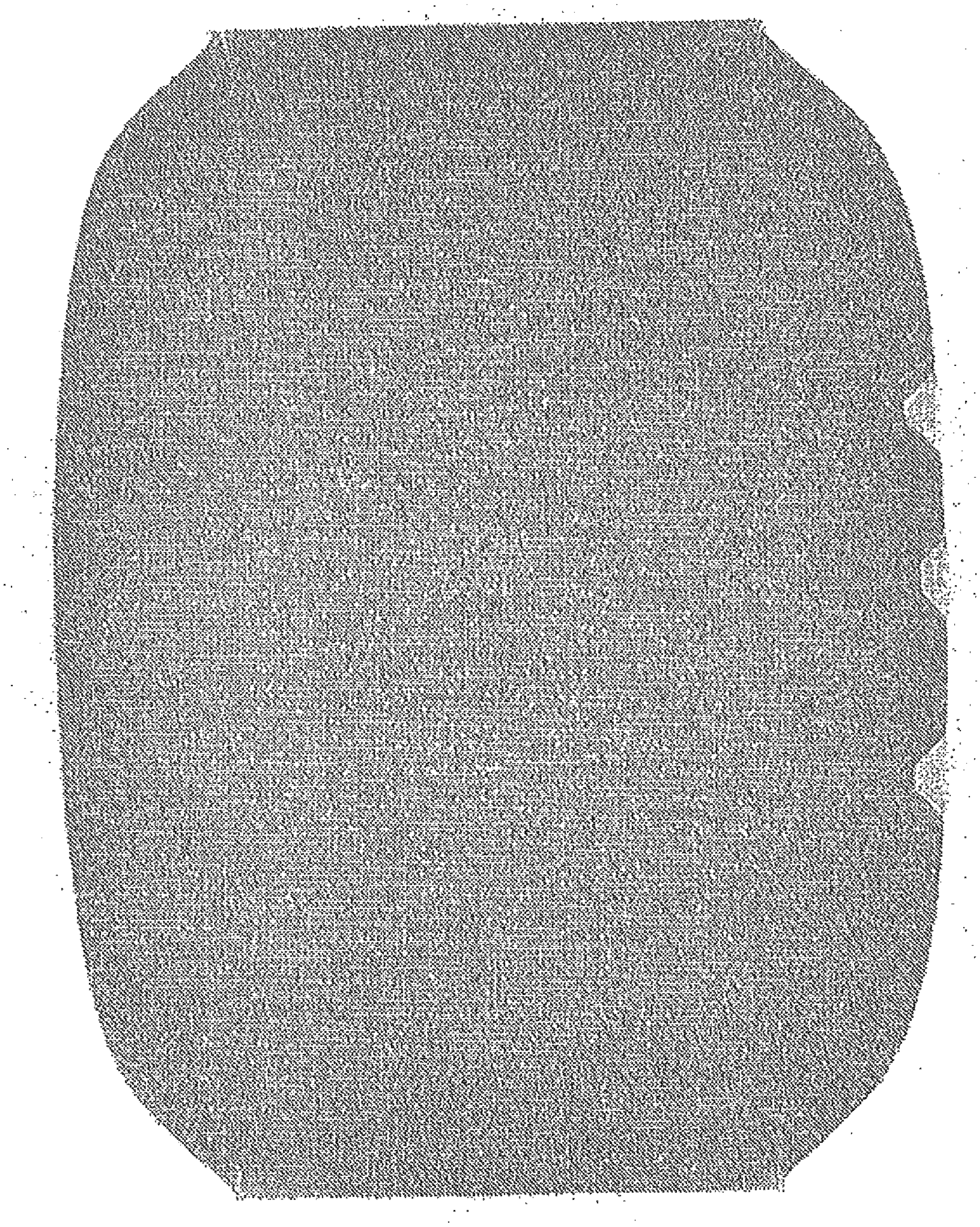

Figure 6A

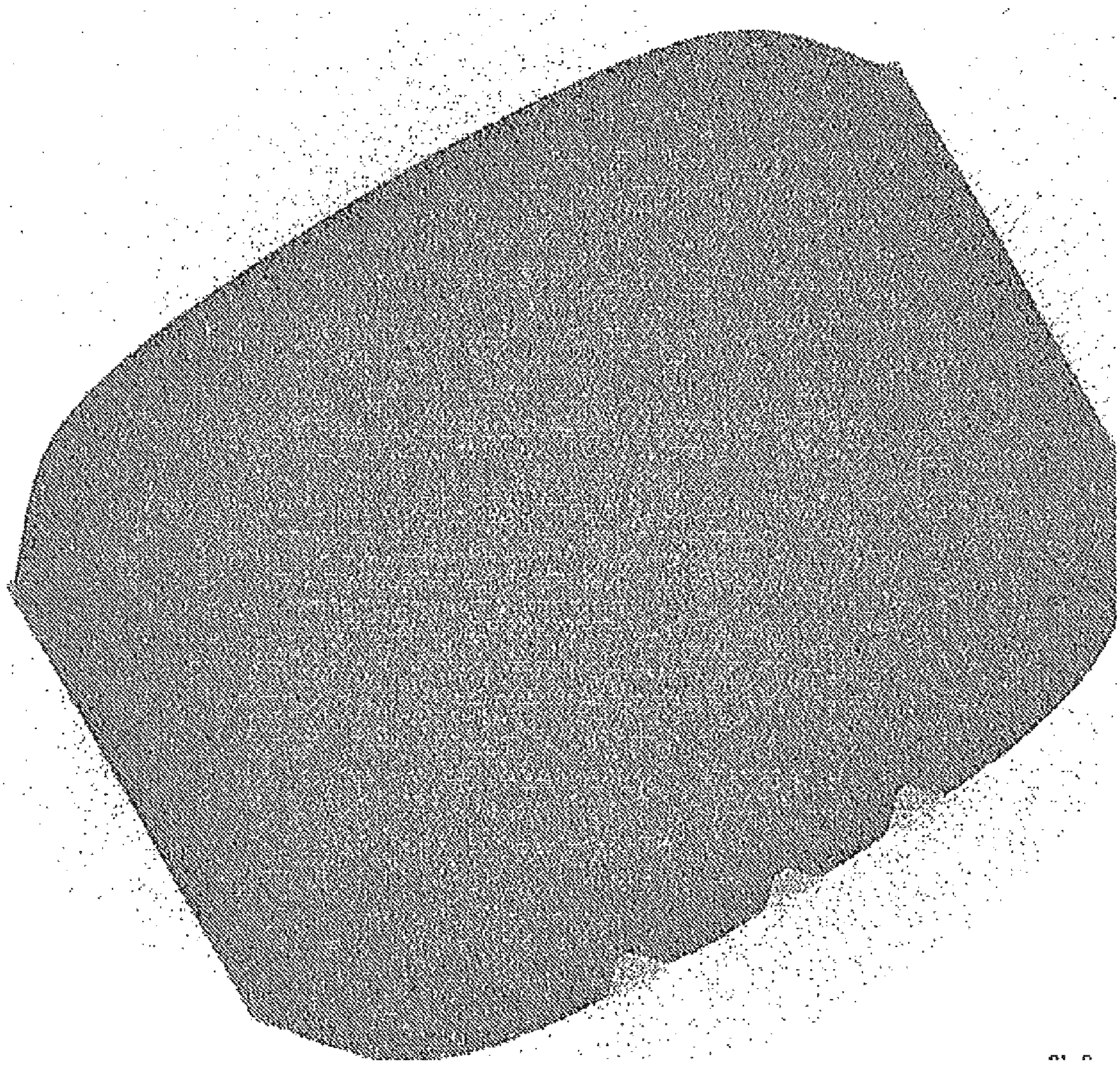

Figure 6B

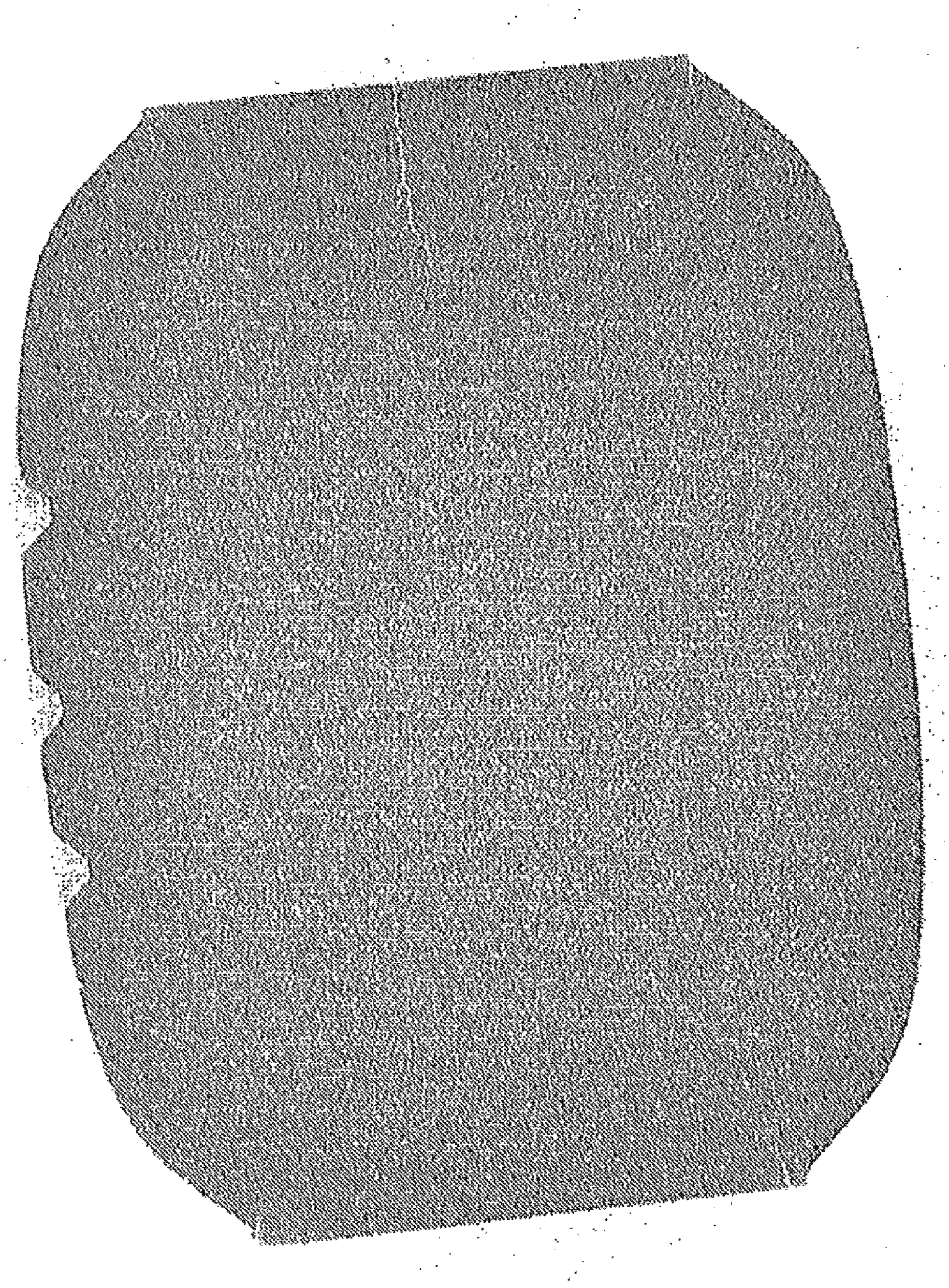

Figure 7A

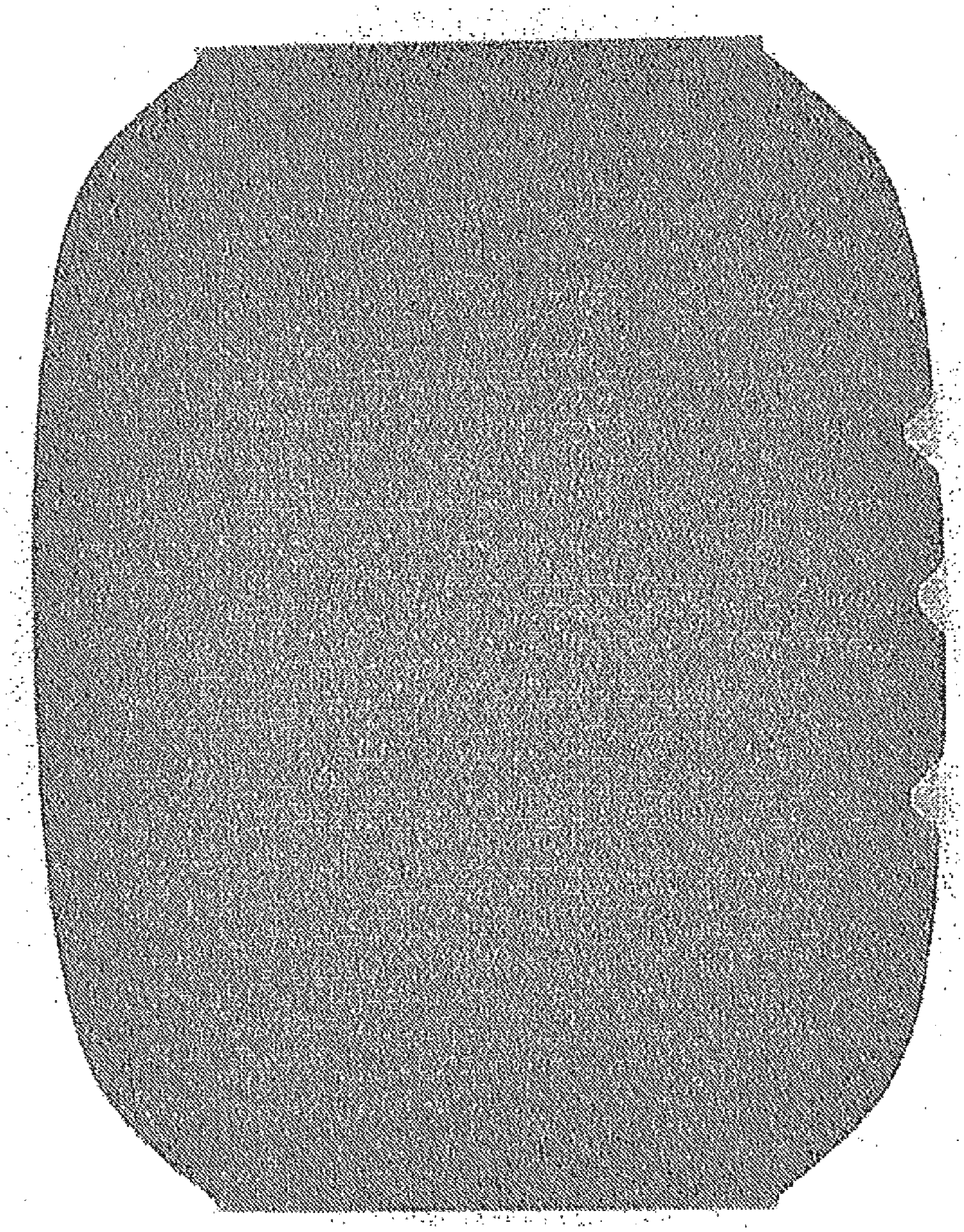

Figure 7B

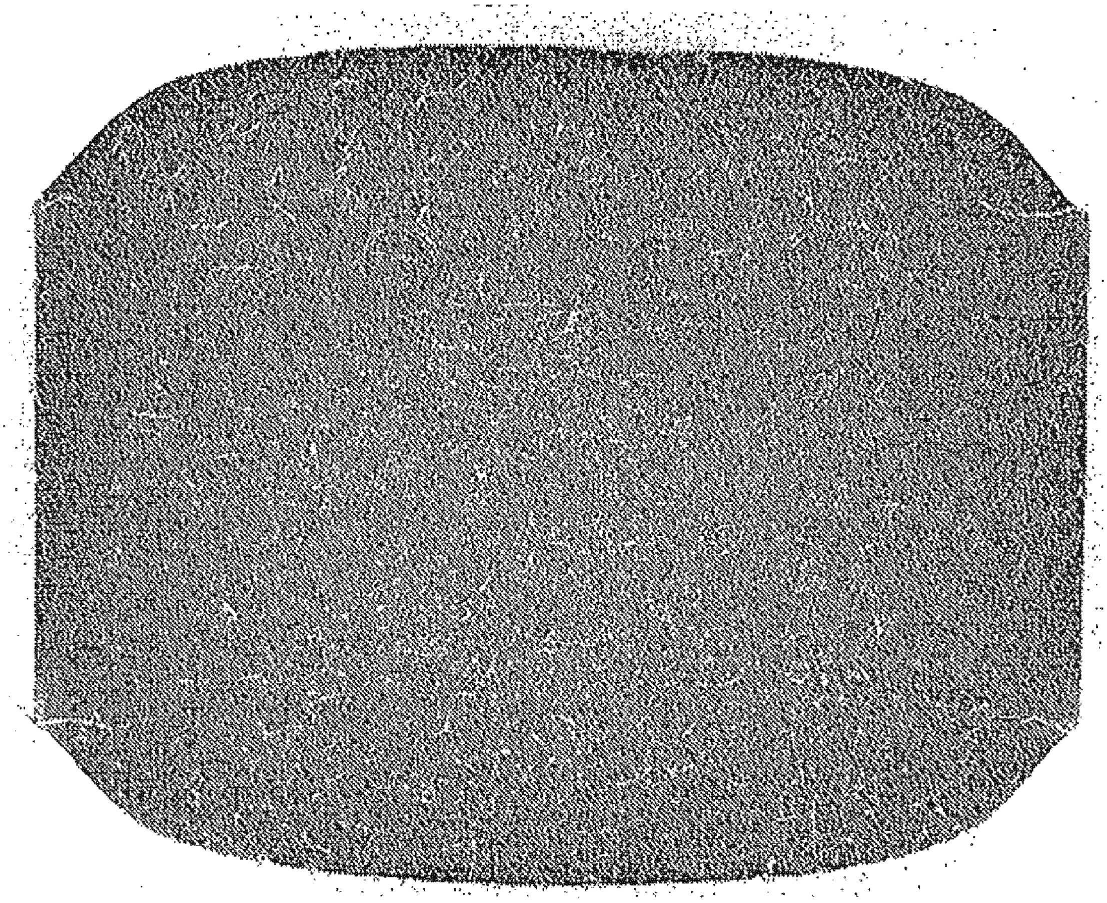

Figure 8A

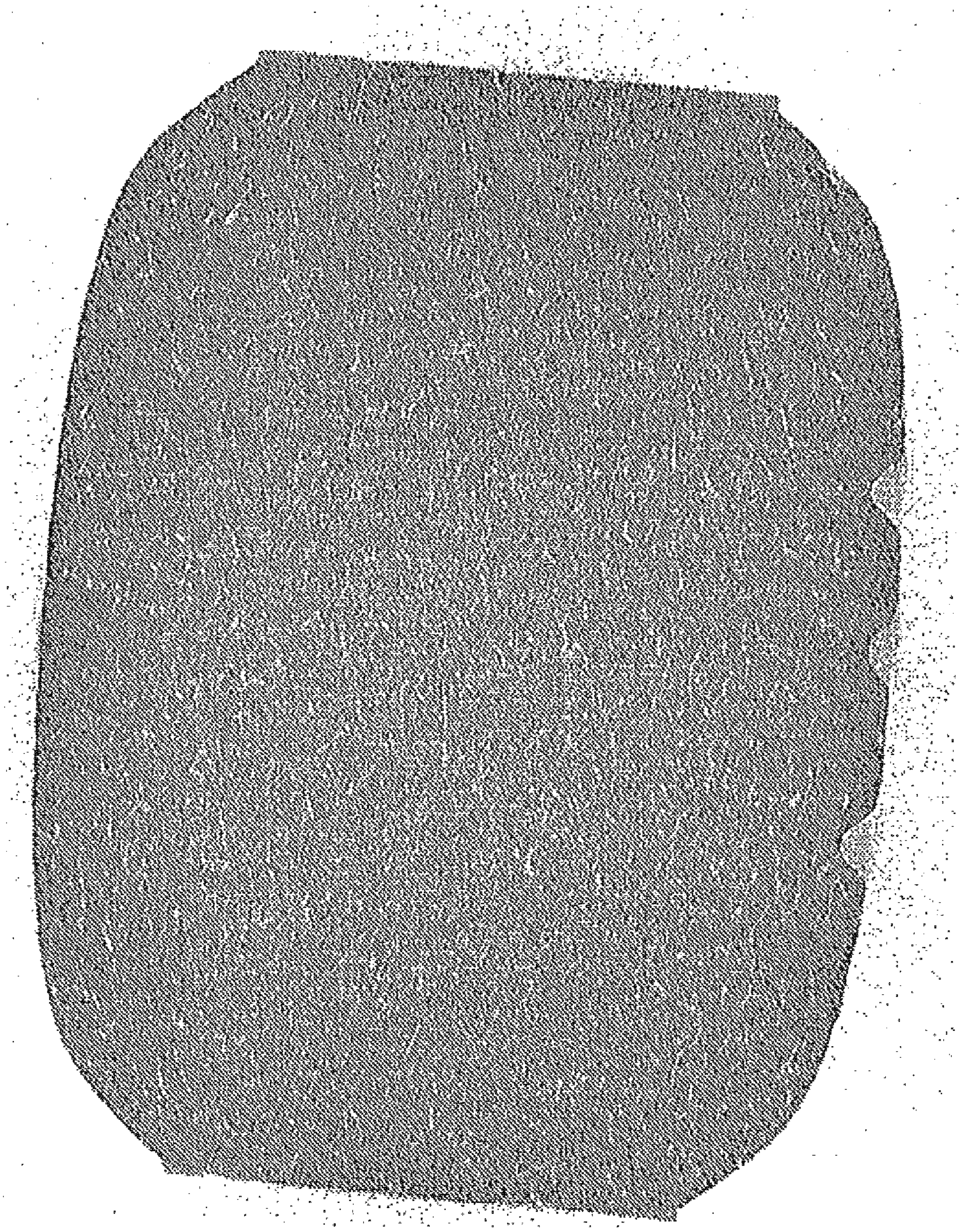

Figure 8B

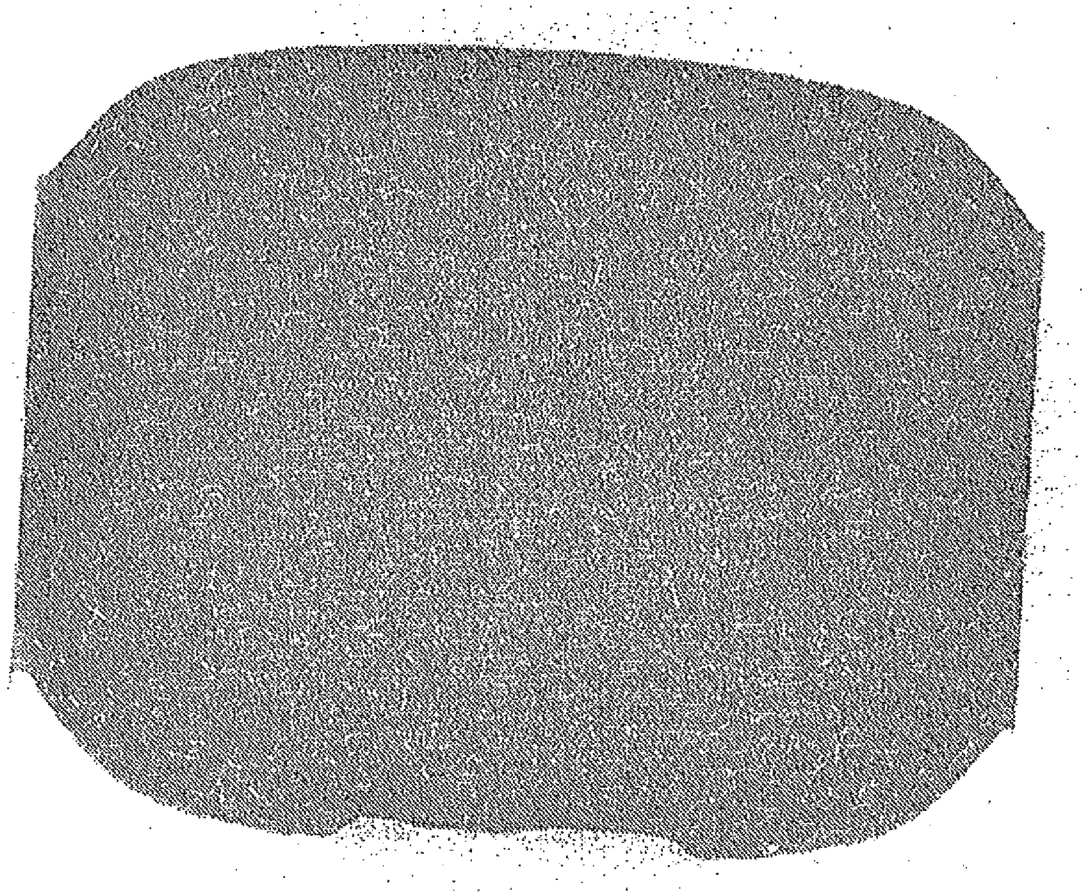

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61032145 B
- US 5609908 A
- US 5700492 A
- US 6764695 B
- US 6964779 B
- US 5541206 A
- US 5914332 A
- US 20050084529 A
- US 6037157 A


### Non-patent literature cited in the description


- Oral Solid Dosage Forms. **E. RUDNIC ; J. SCHWARTZ.** REMINGTON'S PHARMACEUTICAL SCIENCE. 1990, 1633-1658
- Polymer Handbook. John Wiley & Sons, Inc, 1975
- **FIEDLER, H.B.** Encyclopaedia of Excipients. ECV-Editio-Cantor-Verlag, 2002
- Perry's Chemical Engineers' Handbook. McGraw-Hill Book Co, 1984, 20-5420-57
- **MARSHALL.** Atomization and Spray Drying. *50 Chem. Eng. Prog. Monogr. Series 2,* 1954
- Oral Solid Dosage Forms. **E. RUDNIC ; J. SCHWARTZ.** REMINGTON'S PHARMACEUTICAL SCIENCE. 1990, 1641-1645